# EUROPEAN PATENT SPECIFICATION

(11) **EP 1 828 389 B1**
(45) Date of publication and mention of the grant of the patent: **05.10.2011**
(21) Application number: 05814394.2
(22) Date of filing: 30.11.2005
(51) Int. Cl.: C12N 15/85, C40B 40/06, C12Q 1/68, A61K 31/475, A61K 31/704, A61K 31/513, C07H 21/00, A61P 35/00, A61K 33/24

(54) **SPARC PROMOTER MUTATIONS ASSOCIATED WITH DRUG RESISTANCE, AND METHODS ASSOCIATED THEREWITH**
SPARC-PROMOTORMUTATIONEN IM ZUSAMMENHANG MIT ARZNEISTOFFRESISTENZ SOWIE DAMIT VERBUNDENE VERFAHREN
MUTATIONS DU PROMOTEUR DE LA PROTEINE SPARC A UNE RESISTANCE AUX MEDICAMENTS, ET METHODES ASSOCIEES A CES MUTATIONS

(30) Priority: 30.11.2004 US 631414 P
(43) Date of publication of application: 05.09.2007
(73) Proprietor: THE UNIVERSITY OF BRITISH COLUMBIA, Vancouver, British Columbia V6T 1Z3 (CA)
(72) Inventor: TAI, Isabella, T., Vancouver, British Columbia V6Z 3A9 (CA)
(74) Representative: ABG Patentes, S.L.
(86) International application number: PCT/CA2005/001819
(87) International publication number: WO 2006/058425

(56) References cited:
- WO-A1-00/74671
- US-A1- 2003 099 974
- US-A1- 2003 157 073
- GOLDENBERG DANIEL ET AL: "Analysis of differentially expressed genes in hepatocellular carcinoma using cDNA arrays" MOLECULAR CARCINOGENESIS, vol. 33, no. 2, February 2002 (2002-02), pages 113-124, XP002485318 ISSN: 0899-1987
- WANG C-S ET AL: "Overexpression of SPARC Gene in Human Gastric Carcinoma and Its Clinic-Pathologic Significance" BRITISH JOURNAL OF CANCER, LONDON, GB, vol. 91, no. 11, 1 January 2004 (2004-01-01), pages 1924-1930, XP003020536 ISSN: 0007-0920
- LAGAN A L ET AL: "Single-nucleotide polymorphisms in the SPARC gene are not associated with susceptibility to scleroderma" RHEUMATOLOGY (OXFORD), vol. 44, no. 2, 16 November 2004 (2004-11-16), pages 197-201, XP002485319 ISSN: 1462-0324
- DATABASE GENESEQ 25 June 1998 (1998-06-25), JENDRASCHAK ET AL.: "Homo sapiens osteonectin (SPARC) gene, promoter region" XP002485320
- AYCOCK ET AL: 'Development of UV-induced squamous cell carcinomas is suppressed in the absence of SPARC' JOURNAL OF INVESTIGATIVE DERMATOLOGY vol. 123, September 2004, pages 592 - 599, XP008097969
- SHI ET AL: 'Secreted protein acidic, rich in cysteine (SPARC), mediates cellular survival of gliomas through AKT activation' JOURNAL OF BIOLOGICAL CHEMISTRY vol. 279, no. 50, 10 December 2004, pages 52200 - 52209, XP008097976
- JONES ET AL: 'Expression profiling of purified normal human luminal and myoepithelial breast cells: identification of novel prognostic markers for breast cancer' CANCER RESEARCH vol. 64, 01 May 2004, pages 3037 - 3045, XP008097897
- WANG ET AL: 'Overexpression of SPARC gene in human gastric carcinoma and its clinic-pathologic significance' BRITISH JOURNAL OF CANCER vol. 91, 29 November 2004, pages 1924 - 1930, XP003020536
- CHIN ET AL: 'Novel markers for poor prognosis in head and neck cancer' INTERNATIONAL JOURNAL OF CANCER vol. 113, no. 5, 20 February 2005, pages 789 - 797, XP008097963
- HAFNER ET AL: 'A purine-rich sequence in the human BM-40 gene promoter region is a prerequisite for maximum transcription' MATRIX BIOLOGY vol. 14, no. 9, December 1995, pages 733 - 741, XP005161976
- JENDRASCHAKE ET AL: 'Isolation of human promoter regions by Alu repeat consensus-based polymerase chain reaction' GENOMICS vol. 50, no. 1, 15 May 1998, pages 53 - 60, XP004449146
- MASSI ET AL: 'Osteonectin expression correlates with clinical outcome in thin cutaneous malignant melanomas' HUMAN PATHOLOGY vol. 30, no. 3, March 1999, pages 339 - 344, XP008097964
- TOSCANO F ET AL: "p53-mediated upregulation of DcR1 impairs oxaliplatin/TRAIL-induced synergistic anti-tumour potential in colon cancer cells", ONCOGENE, vol. 27, no. 30, July 2008 (2008-07), pages 4161-4171, ISSN: 0950-9232
- LINARDOU HELENA ET AL: "Assessment of somatic k-RAS mutations as a mechanism associated with resistance to EGFR-targeted agents: a systematic review and meta-analysis of studies in advanced non-small-cell lung cancer and metastatic colorectal cancer.", THE LANCET ONCOLOGY OCT 2008 LNKD- PUBMED:18804418, vol. 9, no. 10, October 2008 (2008-10), pages 962-972, ISSN: 1474-5488
- JHAWER MINAXI ET AL: "PIK3CA mutation/PTEN expression status predicts response of colon cancer cells to the epidermal growth factor receptor inhibitor cetuximab", CANCER RESEARCH / AMERICAN ASSOCIATION FOR CANCER RESEARCH, AACR, PHILADELPHIA, PA, vol. 68, no. 6, 15 March 2008 (2008-03-15) , pages 1953-1961, XP002547207, ISSN: 1538-7445, DOI: DOI:10.1158/0008-5472.CAN-07-5659

## Description

### FIELD OF THE INVENTION

The field of the invention relates to the assessment and/or treatment of an animal with a neoplastic condition.

### BACKGROUND OF THE INVENTION

Resistance to chemotherapy is common to many types of cancer and contributes to the high mortality rates in cancer patients. Many factors can play a part in the initial intrinsic resistance to therapy, such as upregulation of efflux pumps from multidrug resistance (MDR) family P-glycoprotein and other MDR proteins (for example multi-drug resistance-associated protein MRP). Such efflux pumps remove chemotherapeutic agents and their metabolites out of cells, thereby decreasing the efficacy of the chemotherapeutic regimen. However, not all cancers that are resistant to chemotherapy have high expression levels of MDR proteins, suggesting that there may be other mechanisms for therapeutic resistance.

Some cancers are particularly resistant to therapies. For example, colorectal cancer (CRC), often accumulates numerous genetic mutations in the progression to tumorigenesis that can also contribute to drug resistance (mutations in p53; loss of DNA mismatch repair (MMR) genes associated with an increased rate of mutation to drug resistance in hereditary non-polyposis colorectal cancer (HNPCC); K-ras mutations; loss of heterozygosity of chromosomes 18q and 22q; and mutations in cell cycle regulatory genes, p21 and p27, is associated with preventing apoptosis in tumors following some chemotherapeutic treatments).

The secreted protein, acidic, rich in cysteine (SPARC), also known as osteonectin, BM-40 and 43K protein, is a type of extracellular protein, termed a matricellular protein, having counter-adhesive properties shown to disrupt cell-matrix interactions (BORNSTEIN P. J. Cell Biol (1995) 130:503-506; SAGE EH. and BORNSTEIN P. J Biol Chem. (1991) 266:14831-4). SPARC is a Ca²⁺-binding glycoprotein, which maps to chromosome 5q31-q33 (SWAROOP A. et al. Genomics (1988) 2:37-47), the genomic sequence is reported to have 10 exons and 9 introns (VILLARREAL XC. et al. Biochemistry (1989) 28(15):6483-6491) with the cDNA extending over ~3 kb. Representative *Homo sapiens* SPARC sequences are listed in GenBank under accession numbers TNM_003118.2 (gi48675809 - mRNA), J03040 (SWAROOP A. *et al.* (1988) *supra*) and 102863 (VILLARREAL XC. *et al.* (1989) *supra*). A representative sequence containing the SPARC promoter, submitted by JENDRASCHAK E. and KAMINSKI WE. (Genomics (1998) 50(1):53-60), is U65081.1 (gi3253140) containing 1370bp. The SPARC promoter region (accession number X82259) was characterized using various promoter constructs in a luciferase assay by HAFNER M. et al. (Matrix Biology (1994) 14:733-741).

SPARC has been associated with bone mineralization (TERMINE JD. et al. Cell (1981) 26:99-105), tissue remodeling (TREMBLE PM. et al. J. Cell Biol. (1993) 121:1433-1444), endothelial cell migration (SAGE EH. et al. J. Cell Biol. (1989) 109:341-356; HASSELAAR P. and SAGE EH. J. Cell Biochem. (1992) 49(3):272-283), apoptosis (SHI Q. et al. J. Biol. Chem. (2004) 279(50):52200-09), angiogenesis (KUPPRION C. et al. J. Cell Biol. (1998) 273(45):29635-29640; VAJKOCZY P. et al. Int. J. Cancer (2000) 87:261-268), scleroderma (LAGAN AL. et al. Rheumatology (2005) 44(2):197-201; ZHOU X. et al. Arthritis & Rheumatism (2002) 46(11):2990-2999) and adipose tissue accumulation (BRADSHAW AD. et al. PNAS (2003) 100(10):6045-6050). Furthermore; SPARC has also been associated with poor overall survival in head and neck cancer (HNSCC) patients (CHIN D. et al. Int. J. Cancer (2005) 113:789-797), poor prognosis in human breast cancer (JONES C. et al. Cancer research (2004) 64:3037-3045), tumor suppressor activity in human ovarian carcinoma cells (MOK SC. et al. Oncogene (1996) 12:1895-1901), development of squamous cell carcinomas in mice (AYCOCK RL. et al. J. Invest. Dermatol. (2004) 123:592-599), inhibition of Lewis lung carcinoma (LLC) cells (BREKKEN RA. et al. J. Clin. Invest. (2003) 111:487-495) and advanced stage human gastric carcinomas (WANG C-S. et al. British Journal of Cancer (2004) 91:1924-1930).

International application, WO 2004/064785, describes methods of sensitizing mammalian cancers to a therapeutic treatment through administration of the SPARC family polypeptides or polynucleotides encoding a SPARC polypeptide. International application, WO 00/4671, describes a method and markers for prognosticating the efficacy of anti-cancer agents by detecting mutant tubulin genes.

### SUMMARY OF THE INVENTION

The invention is defined in and by the appended claims.

This invention is based in part on the surprising discovery that SPARC promoter mutations are associated with therapeutic resistance in a variety of cells and to a variety of therapeutic agents.

This invention is also based in part on the identification the particular mutations in (SPARC) sequence associated with resistance to therapeutic regimens.

In accordance with one aspect of the invention, methods are provided for predicting responsiveness of a subject to a therapeutic regimen, the subject having, or suspected of having a cancer, the method including determining a presence or an absence of one or more a secreted protein, acidic, cysteine-rich (SPARC) promoter mutations in the subject's cancer, wherein said presence or absence of one or more SPARC promoter mutations is indicative of a sensitivity of the subject's cancer to the therapeutic regimen.

In accordance with another aspect of the invention, methods are provided for selecting a group of subjects for determining the efficacy of a therapeutic regimen known or suspected of being useful for the treatment of cancer, the method including determining a genotype at one or more mutation sites in a SPARC promoter for each subject, wherein said genotype is indicative of the subject's sensitivity to a therapeutic regimen. The method may further include, administering the therapeutic regimen to the subjects or a subset of subjects and determining each subject's sensitivity to the therapeutic regimen. The method may further include comparing subject response to the therapeutic regimen based on genotype of the subject.

In accordance with another aspect of the invention, methods are provided for selecting a group of subjects to determine the efficacy of a candidate drug known or suspected of being useful for the treatment of a cancer, the method including detecting one or more mutations in a SPARC sequence for each subject, wherein said one or more mutations are indicative of the subject's response to a therapeutic regimen. The method may also include administering the candidate cancer drug to the subjects or a subset of subjects and determining each subject's response to the therapeutic regimen. The method may also include the additional step of comparing subject response to the candidate drug based on the one or more mutations in the subject's SPARC sequence. Response to the candidate drug may be decided by determining each subject's ability to recover from the cancer or by determining the progression of the cancer in response to the therapeutic regimen.

In accordance with another aspect of the invention, methods are provided for identifying a subject with an increased sensitivity to a therapeutic regimen for treating a cancer in a subject in need thereof, including the step of screening a population of subjects to identify those subjects that have a mutation in their SPARC promoter sequence, wherein the identification of a subject with a mutation is predictive of decreased responsiveness to the therapeutic regimen.

The one or more SPARC promoter mutations, may be selected from one or more of the following mutations relative to SEQ ID NO: 1: (a) 295delG and 301 A>CC; (b) 315insA and 316G>A; (c) 341C>T, 342T>C, 344A>C, 347C>A, 354G>A, 356G>A, 359T>G and 363G>A; (d) 395C>T and 396A>T; (e) 488T>C; (f) 533A>G; (g) 734G>A; (h) 734delG; (i) 769T>A, 771G>C and 774G>C; (j) 784T>C, 786T>G and 788T>A; (k) 793T>G, 796T>A and 800T>C; (l) 820C>A and 824T>A; (m) 864T>A and 867T>A; (n) 895T>G, 899T>A, 901G>A and 904G>A; (o) 901G>A; (p) 901G>A, 904G>A, 929A>G and 934delC; (q) 922T>G, 928T>G and 935T>G; (r) 982T>C, 984T>G, 985G>A, 991G>A, 995G>A, 999C>T and 1000T>G; (s) 1063G>C; (t) 1079G>A; (u) 1085delC, 1086delA, 1087deIG, 1089G>A, 1090T>C, 1091T>C and 1092G>T; (v) 1161C>A, 1162T>C, 1163de1A and II64deIT; and (w) 1215C>T, 1216deIG, 1217deIG, 1218delG, 1219delG, 1220delT, 1221delG, 1222delG, 1223delA, 1224delG, 1225delG, 1226delG, 1227delG, 1228dela, 1229delG, 1230delA, 1231delT, 1232delA, 1233delG, 1234delA, 1235delC, 1236delC and 1237C>T. The therapeutic regimen may be chemotherapy or may further comprise radiotherapy. The therapeutic regimen may include the administration of one or more chemotherapeutic agents. The chemotherapeutic agent may be selected from 5-flurouracil, irinotecan, cisplatin, doxorubicin or combinations thereof.

The one or more SPARC promoter mutations may be indicative of the cancer's resistance to the therapeutic regimen. The method may further include obtaining SPARC promoter information for the subject. The one or more SPARC promoter mutations may be determined using a nucleic acid sample from the subject. The nucleic acid sample may have been obtained from the subject. The obtaining of the nucleic acid sample may be from a biological sample taken from the subject's cancer. The biological sample may be obtained from a biopsy. The biological samples may also be repeatedly obtained at various stages during the therapeutic regimen.

Determining the presence or an absence of one or more SPARC promoter mutations in the subject's cancer may be done using one or more of the following techniques: restriction fragment length analysis; sequencing; micro-sequencing assay; hybridization; invader assay; gene chip hybridization assays; oligonucleotide ligation assay; ligation rolling circle amplification; 5' nuclease assay; polymerase proofreading methods; allele specific PCR; matrix assisted laser desorption ionization time of flight (MALDI-TOF) mass spectroscopy; ligase chain reaction assay; enzyme-amplified electronic transduction; single base pair extension assay; and/or reading sequence data.

The cancer may be selected from one or more of the following: carcinoma; melanoma; sarcoma; lymphoma; and leukaemia. The carcinoma may be selected from the following: squamous cell; adeno; small cell; large cell and combinations thereof. The carcinoma may be derived from a primary tumor located in nasopharygeal tissue, head and neck, lung, esophagus, stomach, intestine, rectum, kidney, urinary tract, prostate, testes, ovary, uterus, cervix, vagina, skin, and endocrine glands. The sarcoma may be selected from the following tissues: muscle; adipose; fibrous; vascular; and neural. The lymphoma may be selected from the following: Hodgkin's Disease; and Non-Hodgkins Lymphoma. The leukaemia may be selected from the following: lymphoid; and myeloid. The cancer may be a colorectal carcinoma, prostate carcinoma, ovarian carcinoma or osteosarcoma. The cancer may be a SPARC inhibited cancer. The SPARC inhibited cancer may be a colorectal carcinoma, prostate carcinoma, ovarian carcinoma, osteosarcoma or uterine sarcoma.

### DETAILED DESCRIPTION OF THE INVENTION

### 1. Definitions

In the description that follows, a number of terms are used extensively, the following definitions are provided to facilitate understanding of the invention.

A "nucleic acid sample" as used herein includes any nucleic acid, and may be a deoxyribonucleotide or ribonucleotide polymer in either single or double-stranded form.

A "purine" is a heterocyclic organic compound containing fused pyrimidine and imidazole rings, and acts as the parent compound for purine bases, adenine (A) and guanine (G). "Nucleotides" are generally a purine (R) or pyrimidine (Y) base covalently linked to a pentose, usually ribose or deoxyribose, where the sugar carries one or more phosphate groups. Nucleic acids are generally a polymer of nucleotides joined by 3' 5' phosphodiester linkages. As used herein "purine" is used to refer to the purine bases, A and G, and more broadly to include the nucleotide monomers, deoxyadenosine-5' - phosphate and deoxyguanosine-5' -phosphate, as components of a polynucleotide chain.

A "pyrimidine" is a single-ringed, organic base that forms nucleotide bases, cytosine (C), thymine (T) and uracil (U). As used herein "pyrimidine" is used to refer to the pyrimidine bases, C, T and U, and more broadly to include the pyrimidine nucleotide monomers that along with purine nucleotides are the components of a polynucleotide chain.

An "allele" is defined as any one or more alternative forms of a given gene. In a diploid cell or organism the members of an allelic pair (i.e. the two alleles of a given gene) occupy corresponding positions (loci) on a pair of homologous chromosomes and if these alleles are genetically identical the cell or organism is said to be "homozygous", but if genetically different the cell or organism is said to be "heterozygous" with respect to the particular gene.

A "gene" is an ordered sequence of nucleotides located in a particular position on a particular chromosome that encodes a specific functional product and may include untranslated and untranscribed sequences in proximity to the coding regions (5' and 3' to the coding sequence). Such non-coding sequences may contain regulatory sequences needed for transcription and translation of the sequence or introns etc. or may as yet to have any function attributed to them beyond the occurrence of the mutation of interest. Numerous examples of SPARC gene sequences, both wild type and mutated, are described herein and provided by SEQ ID NO: 1- 55.

A "genotype" is defined as the genetic constitution of an organism, usually in respect to one gene or a few genes or a region of a gene relevant to a particular context (i.e. the genetic loci responsible for a particular phenotype). For example, as described herein the genotype can refer to the presence or absence of a particular mutation within a SPARC promoter sequence indicative of sensitivity or resistance to a therapeutic regimen.

A "promoter" region is 5' or upstream of the translation start site or ATG start codon.

It will be appreciated by a person of skill in the art that the numerical designations of the positions of mutations within a sequence are relative to the specific sequence. Also the same positions may be assigned different numerical designations depending on the way in which the sequence is numbered and the sequence chosen, as illustrated by the alternative numbering in HAFNER *et al.* (1994) *supra*, in which a SPARC promoter sequence, represented by accession number X82259 has numerous numbering and sequence differences compared to U65081.1 (JENDRASCHAK E. and KAMINSKI WE. (1998) *supra*). Furthermore, sequence variations such as insertions or deletions, may change the relative position and subsequently the numerical designations of particular nucleotides at and around a mutational site.

A nucleotide represented by the symbol M may be either an A or C, a nucleotide represented by the symbol W may be either an T/U or A, a nucleotide represented by the symbol Y may be either an C or T/U, a nucleotide represented by the symbol S may be either an G or C, while a nucleotide represented by the symbol R may be either an G or A, and a nucleotide represented by the symbol K may be either an G or T/U. Similarly, a nucleotide represented by the symbol V may be either A or G or C, as shown in SEQ ID NO:2.

A "mutation" as described herein may be the result of a "single nucleotide polymorphism" (SNP) occurring at a polymorphic site occupied by a single nucleotide, which is the site of variation between allelic sequences. A single nucleotide polymorphism may arise due to substitution of one nucleotide for another at the polymorphic site. A "transition" is the replacement of one purine by another purine or one pyrimidine by another pyrimidine. A "transversion" is the replacement of a purine by a pyrimidine or vice versa. A mutation may also arise from a deletion (represented by "-" or "*del*") of one or more nucleotides or an insertion (represented by "+" or "*ins*") of one or more nucleotides relative to a reference sequence or wildtype (wt) sequence (for example SEQ ID NO:1). Alternatively, a mutation may result in a frameshift of the sequence resulting from a deletion or insertion as described above or from an inversion etc. Additionally, a mutation as described herein, may be a multisite mutation, whereby the mutation is comprised of two or more mutations. Examples of multisite mutations are found as described in (a)-(d), (i)-(n), (p)-(r) and (u)-(w). Specifically, (b) is a combination of 315insA and 316G>A relative to SEQ ID NO:1. Furthermore, it would be appreciated by a person of skill in the art, that an insertion or deletion within a given sequence could alter the relative position and therefore the position number of another mutation within the sequence.

SPARC promoter mutations relative to SEQ ID NO: 1 are shown in TABLE 1 below.

**TABLE 1**

| **Mutation Identifier** | **SPARC MUTATIONS RELATIVE TO SEQ ID NO:1** |
|---|---|
| (a) | 295delG and 301A>CC |
| (b) | 315insA and 316G>A |
| (c) | 341C>T, 342T>C, 344A>C, 347C>A, 354G>A, 356G>A, 359T>G and 363G>A |
| (d) | 395C>T and 396A>T |
| (e) | 488T>C |
| (f) | 533A>G |
| (g) | 734G>A |
| (h) | 734delG |
| (i) | 769T>A, 771G>C and 774G>C |
| (j) | 784T>C, 786T>G and 788T>A |
| (k) | 793T>G, 796T>A and 800T>C |
| (l) | 820C>A and 824T>A |
| (m) | 864T>A and 867T>A |
| (n) | 895T>G, 899T>A, 901G>A and 904G>A |
| (o) | 901G>A |
| (p) | 901G>A, 904G>A, 929A>G and 934delC |
| (q) | 922T>G, 928T>G and 935T>G |
| (r) | 982T>C, 984T>G, 985G>A, 991G>A, 995G>A, 999C>T and 1000T>G |
| (s) | 1063G>C |
| (t) | 1079G>A |
| (u) | 1085delC, 1086delA, 1087delG, 1089G>A, 1090T>C, 1091T>C and 1092G>T |
| (v) | 1161C>A, 1162T>C, 1163delA and 1164deIT |
| (w) | 1215C>T, 1216delG, 1217delG, 1218delG, 1219delG, 1220delT, 1221delG, 1222delG, 1223delA, 1224delG, 1225delG, 1226delG, 1227delG, 1228delA, 1229delG, 1230delA, 1231delT, 1232delA, 1233delG, 1234delA, 1235delC, 1236delC and 1237C>T |

A "polymorphic site" or "polymorphism site" or "polymorphism" or "single nucleotide polymorphism site" (SNP site) as used herein is the locus or position with in a given sequence at which divergence occurs. A "Polymorphism" is the occurrence of two or more forms of a gene or position within a gene (allele), in a population, in such frequencies that the presence of the rarest of the forms cannot be explained by mutation alone. The implication is that polymorphic alleles confer some selective advantage on the host. Preferred polymorphic sites have at least two alleles, each occurring at frequency of greater than 1%, and more preferably greater than 10% or 20% of a selected population. Polymorphic sites may be at known positions within a nucleic acid sequence or may be determined to exist using the methods described herein. Polymorphisms may occur in both the coding regions and the noncoding regions (for example, promoters, enhancers and introns) of genes.

A "SPARC promoter mutation" as used herein refers to any mutation which may be found in a SPARC promoter sequence (for example, SEQ ID NOs: 1-11, but also Accession Not X82259). Such SPARC mutations may be indicative of sensitivity to a therapeutic regimen directed at a cancer whose nucleic acids contain the mutation. For example, SPARC mutations are listed in TABLE 1 ((a)-(w) relative to SEQ ID NO: 1) *supra* and in SEQ ID NOS:2-33.

A "therapeutic regimen" as described herein may be a chemotherapeutic regimen or a radiotherapy regimen or a combination thereof.

As used herein, a "chemotherapeutic regimen" or "chemotherapy" refers to the use of at least one first line chemotherapy agent to destroy cancerous cells, including leukemia and lymphoma. There are a myriad of such first line chemotherapy agents available to a clinician. Chemotherapy agents may be administered to a subject in a single bolus dose, or may be administered in smaller doses over time. A single chemotherapeutic agent may be used (single-agent therapy) or more than one agent may be used in combination (combination therapy). Chemotherapy may be used alone to treat some types of cancer. Alternatively, chemotherapy may be used in combination with other types of treatment, for example, radiotherapy or "alternative therapies (for example immunotherapy) as described herein.

As used herein, a first line "chemotherapeutic agent" or first line chemotherapy is a medicament that may be used to treat cancer, and generally has the ability to kill cancerous cells directly. Examples of first line chemotherapeutic agents include alkylating agents, antimetabolites, natural products, hormones and antagonists, and miscellaneous agents. Examples of alternate names are indicated in brackets. Examples of alkylating agents include nitrogen mustards such as mechlorethamine, cyclophosphamide, ifosfamide, melphalan (L-sarcolysin) and chlorambucil; ethylenimines and methylmelamines such as hexamethylmelamine and thiotepa; alkyl sulfonates such as busulfan; nitrosoureas such as carmustine (BCNU), semustine (methyl-CCNU), lomustine (CCNU) and streptozocin (streptozotocin); DNA synthesis antagonists such as estramustine phosphate; and triazines such as dacarbazine (DTIC, dimethyltriazenoimidazolecarboxamide) and temozolomide . Examples of antimetabolites include folic acid analogs such as methotrexate (amethopterin); pyrimidine analogs such as fluorouracin (5-fluorouracil, 5-FU, 5FU), floxuridine (fluorodeoxyuridine, FUdR), cytarabine (cytosine arabinoside) and gemcitabine; purine analogs such as mercaptopurine (6-mercaptopurine, 6-MP), thioguanine (6-thioguanine, TG) and pentostatin (2'-deoxycoformycin, deoxycoformycin), cladribine and fludarabine; and topoisomerase inhibitors such as amsacrine. Examples of natural products include vinca alkaloids such as vinblastine (VLB) and vincristine; taxanes such as paclitaxel and docetaxel (Taxotere); epipodophyllotoxins such as etoposide and teniposide; camptothecins such as topotecan and irinotecan; antibiotics such as dactinomycin (actinomycin D), daunorubicin (daunomycin, rubidomycin), doxorubicin, bleomycin, mitomycin (mitomycin C), idarubicin, epirubicin; enzymes such as L-asparaginase; and biological response modifiers such as interferon alpha and interlelukin 2. Examples of hormones and antagonists include luteinising releasing hormone agonists such as buserelin; adrenocorticosteroids such as prednisone and related preparations; progestins such as hydroxyprogesterone caproate, medroxyprogesterone acetate and megestrol acetate; estrogens such as diethylstilbestrol and ethinyl estradiol and related preparations; estrogen antagonists such as tamoxifen and anastrozole; androgens such as testosterone propionate and fluoxymesterone and related preparations; androgen antagonists such as flutamide and bicalutamide; and gonadotropin-releasing hormone analogs such as leuprolide. Examples of miscellaneous agents include thalidomide; platinum coordination complexes such as cisplatin (cis-DDP), oxaliplatin and carboplatin; anthracenediones such as mitoxantrone; substituted ureas such as hydroxyurea; methylhydrazine derivatives such as procarbazine (N-methylhydrazine, MIH); adrenocortical suppressants such as mitotane (o,p'-DDD) and aminoglutethimide; RXR agonists such as bexarotene; and tyrosine kinase inhibitors such as imatinib. Alternate names and trade-names of these and additional examples of chemotherapeutic agents, and their methods of use including dosing and administration regimens, will be known to a physician versed in the art, and may be found in, for example "The Pharmacological basis of therapeutics", 10th edition. HARDMAN HG., LIMBIRD LE. editors. McGraw-Hill, New York, and in "Clinical Oncology", 3rd edition. Churchill Livingstone/ Elsevier Press, 2004. ABELOFF, MD. editor.

As used herein, the term "radiotherapeutic regimen" or "radiotherapy" refers to the administration of radiation to kill cancerous cells. Radiation interacts with various molecules within the cell, but the primary target, which results in cell death is the deoxyribonucleic acid (DNA). However, radiotherapy often also results in damage to the cellular and nuclear membranes and other organelles. DNA damage usually involves single and double strand breaks in the sugar-phosphate backbone. Furthermore, there can be cross-linking of DNA and proteins, which can disrupt cell function. Depending on the radiation type, the mechanism of DNA damage may vary as does the relative biologic effectiveness. For example, heavy particles (i.e. protons, neutrons) damage DNA directly and have a greater relative biologic effectiveness. Whereas, electromagnetic radiation results in indirect ionization acting through short-lived, hydroxyl free radicals produced primarily by the ionization of cellular water. Clinical applications of radiation consist of external beam radiation (from an outside source) and brachytherapy (using a source of radiation implanted or inserted into the patient). External beam radiation consists of X-rays and/or gamma rays, while brachytherapy employs radioactive nuclei that decay and emit alpha particles, or beta particles along with a gamma ray.

Whether direct or indirect damage from radiotherapy results in the loss of cell division and therefore cell growth. Accordingly, many radiation damaged cells are only detected when they try to undergo cell division and associated processes (for example replication). However, some cells will actually make it through a few rounds of cell division before the damage results in cell death, while others may be recognized a damaged sooner and undergo apoptosis before the cell even attempts division. Therefore depending on the type of radiation applied, the intensity of the radiation, the cell type and other factors, it may be apparent that a cell has been damaged by a radiotherapeutic regimen or it may not, even with histologic review.

Radiotherapy may further be used in combination chemotherapy, with the chemotherapeutic agent acting as a radiosensitizer. The specific choice of radiotherapy suited to an individual patient may be determined by a physician or oncologist, taking into consideration the tissue and stage of the cancer. Examples of radiotherapy approaches to various cancers may be found in, for example. "Clinical Oncology", 3rd edition. Churchill Livingstone/ Elsevier Press, 2004. ABELOFF, MD. editor.

As used herein the term "alternative therapeutic regimen" or "alternative therapy" (not a first line chemotherapeutic regimen as described above) may include for example, SPARC polypeptide (as described in WO 2004/064785), receptor tyrosine kinase inhibitors (for example Iressa™ (gefitinib), Tarceva™ (erlotinib), Erbitux™ (cetuximab), imatinib mesilate (Gleevec™), proteosome inhibitors (for example bortezomib, Velcade™); VEGFR2 inhibitors such as PTK787 (ZK222584), aurora kinase inhibitors (for example ZM447439); mammalian target of rapamycin (mTOR) inhibitors, cyclooxygenase-2 (COX-2) inhibitors, rapamycin inhibitors (for example sirolimus, Rapamune™); farnesyltransferase inhibitors (for example tipifarnib, Zarnestra); matrix metalloproteinase inhibitors (for example BAY 12-9566; sulfated polysaccharide tecogalan); angiogenesis inhibitors (for example Avastin™ (bevacizumab); analogues of fumagillin such as TNP-4; carboxyaminotriazole; BB-94 and BB-2516; thalidomide; interleukin-12; linomide; peptide fragments; and antibodies to vascular growth factors and vascular growth factor receptors); platelet derived growth factor receptor inhibitors, protein kinase C inhibitors, mitogen-activated kinase inhibitors, mitogen-activated protein kinase kinase inhibitors, Rouse sarcoma virus transforming oncogene (SRC) inhibitors, histonedeacetylase inhibitors, small hypoxia-inducible factor inhibitors, hedgehog inhibitors, and TGF-β signalling inhibitors. Furthermore, an immunotherapeutic agent would also be considered an alternative therapeutic regimen. For example, serum or gamma globulin containing preformed antibodies; nonspecific immunostimulating adjuvants; active specific immunotherapy; and adoptive immunotherapy. In addition, alternative therapies may include other biological-based chemical entities such as polynucleotides, including antisense molecules, polypeptides, antibodies, gene therapy vectors and the like. Such alternative therapeutics may be administered alone or in combination, or in combination with other therapeutic regimens described herein. Alternate names and trade-names of these agents used in alternative therapeutic regimens and additional examples of agents used in alternative therapeutic regimens, and their methods of use including dosing and administration regimens, will be known to a physician versed in the art. Furthermore, methods of use of chemotherapeutic agents and other agents used in alternative therapeutic regimens in combination therapies, including dosing and administration regimens, will also be known to a physician versed in the art.

The detection of nucleic acids in a sample may rely on the technique of specific nucleic acid hybridization in which the oligonucleotide is annealed under conditions of "high stringency" to nucleic acids in the sample, and the successfully annealed oligonucleotides are subsequently detected (see for example Spiegelman, S., Scientific American, Vol. 210, p. 48 (1964)). Hybridization under high stringency conditions primarily depends on the method used for hybridization, the oligonucleotide length, base composition and position of mismatches (if any). High stringency hybridization is relied upon for the success of numerous techniques routinely performed by molecular biologists, such as high stringency PCR, DNA sequencing, single strand conformational polymorphism analysts, and in situ hybridization. In contrast to northern and Southern hybridizations, these techniques are usually performed with relatively short probes (e.g., usually about 16 nucleotides or longer for PCR or sequencing and about 40 nucleotides or longer for in situ hybridization). The high stringency conditions used in these techniques are well known to those skilled in the art of molecular biology, and examples of them can be found, for example, in AUSUBEL et al., Current Protocols in Molecular Biology, John Wiley & Sons, New York, N.Y., 1998.

A representative of a *Homo sapiens* SPARC promoter gene sequence is listed in GenBank under accession number U65081 (SEQ ID NO:1). Polymorphic sites in SPARC promoter sequence are identified by their variant (i.e. M, W, Y, S, R, K or V) in SEQ ID NO:2.

**TABLE 2 - Primers**

| **SEQ ID** | **Primer Sequence** | **Nucleotide Position** | **Primer name** |
|---|---|---|---|
| 34 | | 35-57 of SEQ ID NO: 9 | Set 1 fwd |
| 35 | | 546-567 of SEQ ID NO: 9 (on opposite strand) | Set 1 rev |
| 36 | | 431-452 of SEQ ID NO: 9 | Set 2 fwd |
| 37 | | 1360-1380 of SEQ ID NO: 9(on opposite strand) | Set 2 rev |
| 38 | GTCATCCCTC CAGGCC | 1126-1141 of SEQ ID NO: 9 | Set 3 fwd |
| 39 | | 2905-2924 of SEQ ID NO: 9 (on opposite strand) | Set 3 rev |
| 40 | | 430-452 of SEQ ID NO: 9 | SPARC fwd (with 5' M13 tag in lower case) |
| 41 | | 1359-1379 of SEQ ID NO: 9 (on opposite strand) | SPARC rev (with 5' M13 tag in lower case) |
| 42 | | 1338-1354 of SEQ ID NO: 9 | sp-seq-1-F (with 5' M13 tag in lower case) |
| 43 | | 2140-2158 of X82259 (on opposite strand) | sp seq 1-R (with M13 tag in lower case) |
| 44 | | 430-452 of SEQ ID NO: 9 | sp seq-up-1-F (with 5' M13 tag in lower case) |
| 45 | | 1360 -1380 of SEQ ID NO: 9 (on opposite strand) | sp seq-up-1-R (with 5' M13 tag in lower case) |
| 46 | | 2097-2116 of X82259 | sp seq 2-F (with 5' M13 tag in lower case) |
| 47 | | Intronic | sp seq 2-R (with 5' M13 tag in lower case) |
| 48 | | 31-52 of X82259 | sp-seq up-0-F (with 5' M13 tag in lower case) |
| 49 | | 546-567 of SEQ ID NO: 9 (on opposite strand) | sp-seq up-0-R (with 5' M13 tag in lower case) |
| 50 | | 1126-1141 of SEQ ID NO: 9 | sp-seq up-2-F (with 5' M13 tag in lower case) |
| 51 | | 1527-1512 of X82259 | sp-seq up-2-R (with 5' M13 tag in lower case) |
| 52 | | (1553-1575 of SEQ ID NO: 1) | SPARC fwd (TAI 2005) |
| 53 | | 1901-1926 of SEQ ID NO: 1 (opposite strand) | SPARC rev (TAI 2005) |
| 54 | | Not SPARC | GAPDH fwd (TAI 2005) |
| 55 | | Not SPARC | GAPDH rev (TAI 2005) |

### Sparc Promoter Sequencing Primers

Sequencing primers corresponding to SEQ ID NO: 34 and SEQ ID NO: 35 were used to obtain sequence information on both strands of the SPARC promoter (SEQ ID NO: 1) for the region between nucleotide 1 and nucleotide 425.

Sequencing primers corresponding to SEQ ID NO: 36 and SEQ ID NO: 37 were used to obtain sequence information on both strands of the SPARC promoter (SEQ ID NO: 1) for the region between nucleotide 333 and nucleotide 1241.

Sequencing primers corresponding to SEQ ID NO: 38 and SEQ ID NO: 39 were used to obtain sequence information on both strands of the SPARC promoter (SEQ ID NO: 1) for the region between nucleotide 1024 and nucleotide 1370.

The sequences shown in TABLE 3 below (SEQ ID NOS: 11-33) show SPARC promoter mutations (underligned) with flanking sequences. The numbering associated with these sequences represents the mutation positions relative to SEQ ID NO:1 (wild type SPARC promoter sequence).

**TABLE 3**

| **SPARC PROMOTER Mutations** | **SEQ ID NO:** | **MUTATION(S) WITH FLANKING SEQUENCE** |
|---|---|---|
| 295deIG and 301A>CC | 11 | |
| 315insA and 316G>A | 12 | |
| 341C>T, 342T>C, 344A>C, 347C>A, 354G>A, 356G>A, 359T>G and 363G>A | 13 | |
| 395C>T and 396A>T | 14 | |
| 488T>C | 15 | |
| 533A>G | 16 | |
| | | |
| 734G>A , | 17 | |
| 734delG | 18 | |
| 769T>A. 771 G>C and 774G>C | 19 | |
| 784T>C, 786T>G and 788T>A | 20 | |
| 793T>G, 796T>A and 800T>C | 21 | |
| 820C>A and 824T>A | 22 | |
| 864T>A and 867T>A | 23 | |
| 895T>G, 899T>A, 901 G>A and 904G>A | 24 | |
| 901G>A | 25 | |
| 901G>A, 904G>A, 929A>G and 934delC | 26 | |
| 922T>G, 928T>G and 935T>G | 27 | |
| 982T>C, 984T>G, 985G>A, 991G>A, 995G>A, 999C>T and 1000T>G | 28 | |
| 1063G>C | 29 | |
| 1079G>A | 30 | |
| 1085delC, 1086delA, | 31 | |
| 1087delG, 1089G>A, 1099T>C, 109lT>C and 1092G>T | | |
| 1161C>A, 1162T>C, 1163delA and 1164delT | 32 | |
| 1215C>T, 1216delG, 1217delG, 1218delG, 1219delG, 1220delT, 1221delG, 1222delG, 1223delA, 1224delG, 1225delG, 1226delG, 1227delG, 1228delA, 1229delG, 1230delA, 1231delT, 1232delA, 1233delG, 1234delA, 1235delC, 1236delC and 1237C>T | 33 | |

The Sequences given in TABLE 3 (SEQ ID NO: 11-33) above and in SEQ ID NO:1-10 may be useful to a person of skill in the art in the design of further primers, and probes or other oligonucleotides or peptide nucleic acids for the identification of SPARC mutations as described herein.

"Oligonucleotides" as used herein are variable length nucleic acids, which may be useful as probes, primers and in the manufacture of microarrays (arrays) for the detection and/or amplification of specific nucleic acids. Such DNA or RNA strands may be synthesized by the sequential addition (5'-3' or 3'-5') of activated monomers to a growing chain which may be linked to an insoluble support. Numerous methods are known in the art for synthesizing oligonucleotides for subsequent individual use or as a part of the insoluble support, for example in arrays (BERNFIELD MR. and ROTTMAN FM. J. Biol. Chem. (1967) 242(18):4134-43; SULSTON J. et al. PNAS (1968) 60(2):409-415; GILLAM S. et al. Nucleic Acid Res.(1975) 2(5):613-624; BONORA GM. et al. Nucleic Acid Res.(1990) 18(11):3155-9; LASHKARI DA. et al. PNAS (1995) 92(17):7912-5; MCGALL G. et al. PNAS (1996) 93(24):13555-60; ALBERT TJ. et al. Nucleic Acid Res.(2003) 31(7):e35; GAO X. et al. Biopolymers (2004) 73(5):579-96; and MOORCROFT MJ. et al. Nucleic Acid Res.(2005) 33(8):e75). In general, oligonucleotides are synthesized through the stepwise addition of activated and protected monomers under a variety of conditions depending on the method being used. Subsequently, specific protecting groups may be removed to allow for further elongation and subsequently and once synthesis is complete all the protecting groups may be removed and the oligonucleotides removed from their solid supports for purification of the complete chains if so desired.

"Peptide nucleic acids" (PNA) as used herein refer to modified nucleic acids in which the sugar phosphate skeleton of a nucleic acid has been converted to an N-(2-aminoethyl)-glycine skeleton. Although the sugar-phosphate skeletons of DNA/RNA are subjected to a negative charge under neutral conditions resulting in electrostatic repulsion between complementary chains, the backbone structure of PNA does not inherently have a charge. Therefore, there is no electrostatic repulsion. Consequently, PNA has a higher ability to form double strands as compared with conventional nucleic acids, and has a high ability to recognize base sequences. Furthermore, PNAs are generally more robust than nucleic acids.

A "vector" as used herein includes any means for delivery of a nucleic acid sample to a cell. For example, a vector may include a plasmid, artificial chromosome etc.

A "cancer" is defined as including cancers in which mutations can be detected in accordance with the methods described herein and can include but, are not limited to, carcinomas, mealomas, sarcomas, lymphomas, and leukaemias. Carcinomas may include, without limitation, squamous cell, adeno, small cell and large cell carcinomas, and combinations thereof. Carcinomas may have their primary tumor located, for example in the nasopharygeal tissue, head and neck, lung, esophagus, stomach, intestine, rectum, kidney, urinary tract, prostate, testes, ovary, uterus, cervix, vagina, skin, and endocrine glands or may be multicentric. Sarcomas may be, for example of muscle, adipose, fibrous, vascular, or neural origin. Lymphomas may include, without, limitation, for example Hodgkin's Disease and Non-Hodgkins Lymphoma. Leukaemias may be, for example, of lymphoid or myeloid cell origin and may be acute or chronic in presentation and/or course. Furthermore, a cancer may be a "SPARC inhibited cancer", whereby the cancer decreases SPARC expression to facilitate growth of the cancer. The cancer may, for example, also be selected from colorectal carcinomas, prostate carcinomas, ovarian carcinomas, osteosarcomas or combinations thereof.

A cancer may be described as "sensitive to" or "resistant to" a given therapeutic regimen based on the ability of the regimen to kill cancer cells or decrease tumor size, reduce overall cancer growth (i.e. through reduction of angiogenesis), and or inhibit metastasis. Cancer cells that are resistant to a therapeutic regimen would not respond to the regimen and may continue to proliferate. Whereas cancer cells that are sensitive to a therapeutic regimen are those that do respond to the regimen resulting in cell death, a reduction in tumor size, reduced overall growth or inhibition of metastasis. Monitoring of a response may be accomplished by numerous pathological, clinical and imaging methods as described herein and known to persons of skill in the art.

**TABLE 4. below shows correlation of SPARC mutations with values representing resistance (R) or sensitivity (S) to a therapeutic regimen for the treatment of cancer.**

| **Mutation Identifier** | **SPARC MUTATIONS RELATIVE TO SEQ ID NO:1** | **RESPONSE TO THERAPY** |
|---|---|---|
| **(a)** | 295delG and 301A>CC | R |
| **(b)** | 315insA and 316G>A | R |
| **(c)** | 341C>T, 342T>C, 344A>C, 347C>A, 354G>A, 356G>A, 359T>G and 363G>A | R |
| **(d)** | 395C>T and 396A>T | R |
| **(e)** | 488T>C | R |
| **(f)** | 533A>G | R |
| **(g)** | 734G>A | R |
| **(b)** | 734delG | R |
| **(i)** | 769T>A, 771 G>C and 774G>C | R |
| **(j)** | 784T>C, 786T>G and 788T>A | R |
| **(k)** | 793T>G, 796T>A and 800T>C | R |
| **(l)** | 820C>A and 824T>A | R |
| **(m)** | 864T>A and 867T>A | R |
| **(n)** | 895T>G, 899T>A. 901G>A and 904G>A | R |
| **(o)** | 901G>A | R |
| **(p)** | 901 G>A, 904G>A, 929A>G and 934delC | R |
| **(q)** | 922T>G, 928T>G and 935T>G | R |
| **(r)** | 982T>C, 984T>G, 985G>A, 991G>A, 995G>A, 999C>T and 1000T>G | R |
| **(s)** | 1063G>C | R |
| **(t)** | 1079G>A | R |
| **(u)** | 1085delC, 1086delA, 1087delG, 1089G>A, 1090T>C, 1091T>C and 1092G>T | R |
| **(v)** | 1161C>A, 1162T>C, 1163delA and 1164delT | R |
| **(w)** | 1215C>T, 1216delG, 1217delG, 1218delG, 1219delG, 1220delT, 1221delG, 1222delG, 1223delA, 1224delG, 1225delG, 1226delG, 1227delG, 1228delA, 1229delG, 1230delA, 1231delT, 1232delA, 1233delG, 1234delA, 1235delC, 1236delC and 1237C>T | R |
| **(x)** | Wild type | S |

Sensitive (S); Resistant (R).

### 2.A. General Methods

Once a subject is identified as being at risk for developing or having a cancer or a SPARC-inhibited cancer, genetic sequence information may be obtained from the subject. Various methods for obtaining biological samples from a subject that contain genetic material, are known in the art. For example, tissue samples may be obtained by curettage, needle aspiration biopsy or needle (core) biopsy, incisional biopsy for sampling a tumor, or excisional biopsy, which may entail total removal of the tissue of interest. Alternatively, other bodily samples that contain genetic material, such as hair, sputum, urine, stool, semen or blood may be collected using methods known in the art.

Genetic sequence information may be obtained from a biological sample containing genetic material in numerous different ways, particularly, genetic material containing the sequence or sequences of interest. Many methods are known in the art for extracting genetic material from biological samples. There are many known methods for the separate isolation of DNA and RNA from biological samples. Typically, DNA may be isolated from a biological sample when first the sample is lysed and then the DNA is isolated from the lysate according to any one of a variety of multi-step protocols, which can take varying lengths of time. DNA isolation methods may involve the use of phenol

(SAMBROOK, J. et al. "Molecular Cloning", Vol. 2, pp. 9.14-9.23, Cold Spring Harbor Laboratory Press (1989) and AUSUBEL, FM. et al., "Current Protocols in Molecular Biology", Vol. 1, pp. 2.2.1-2.4.5, John Wiley & Sons, Inc. (1994)). Typically, a biological sample is lysed in a detergent solution and the protein component of the lysate is digested with proteinase for 12-18 hours. Next, the lysate is extracted with phenol to remove most of the cellular components, and the remaining aqueous phase is processed further to isolate DNA. In another method, described in VAN NESS *et al.* (U.S. Pat. # 5,130,423), non-corrosive phenol derivatives are used for the isolation of nucleic acids. The resulting preparation is a mix of RNA and DNA.

Other methods for DNA isolation utilize non-corrosive chaotropic agents. These methods, which are based on the use of guanidine salts, urea and sodium iodide, involve lysis of a biological sample in a chaotropic aqueous solution and subsequent precipitation of the crude DNA fraction with a lower alcohol. The final purification of the precipitated, crude DNA fraction can be achieved by any one of several methods, including column chromatography (Analects, (1994) Vol 22, No. 4, Pharmacia Biotech), or exposure of the crude DNA to a polyanion-containing protein as described in KOLLER (U.S. Pat. # 5,128,247).

Yet another method of DNA isolation, which is described by BOTWELL, DDL. (Anal. Biochem. (1987) 162:463-465) involves lysing cells in 6M guanidine hydrochloride, precipitating DNA from the lysate at acid pH by adding 2.5 volumes of ethanol, and washing the DNA with ethanol.

Numerous other methods are known in the art to isolate both RNA and DNA, such as the one described by CHOMCZYNSKI (U.S. Pat. # 5,945,515), whereby genetic material can be extracted efficiently in as little as twenty minutes. EVANS and HUGH (U.S. Pat. # 5,989,431) describe methods for isolating DNA using a hollow membrane filter.

Once a subject's genetic material has been obtained from the subject it may then be further be amplified by Reverse Transcription Polymerase Chain Reaction (RT-PCR), Polymerase Chain Reaction (PCR), Transcription Mediated Amplification (TMA), Ligase chain reaction (LCR), Nucleic Acid Sequence Based Amplification (NASBA) or other methods known in the art, and then further analyzed to detect or determine the presence or absence of one or more polymorphisms or mutations in the sequence of interest, provided that the genetic material obtained contains the sequence of interest. Particularly, a person may be interested in determining the presence or absence of a mutation in the SPARC promoter sequence , as described in TABLE 1. The sequence of interest may also include other mutations, or may also contain some of the sequence surrounding the mutation of interest.

Detection or determination of a nucleotide identity, or the presence of one or more single nucleotide polymorphism(s) (SNP typing), may be accomplished by any one of a number methods or assays known in the art. Many DNA typing methodologies are useful detection of SNPs. The majority of SNP genotyping reactions or assays can be assigned to one of four broad groups (sequence-specific hybridization, primer extension, oligonucleotide ligation and invasive cleavage). Furthermore, there are numerous methods for analyzing/detecting the products of each type of reaction (for example, fluorescence, luminescence, mass measurement, electrophoresis, etc.). Furthermore, reactions can occur in solution or on a solid support such as a glass slide, a chip, a bead, etc.

In general, sequence-specific hybridization involves a hybridization probe, which is capable of distinguishing between two DNA targets differing at one nucleotide position by hybridization. Usually probes are designed with the polymorphic base in a central position in the probe sequence, whereby under optimized assay conditions only the perfectly matched probe target hybrids are stable and hybrids with a one base mismatch are unstable. A strategy which couples detection and sequence discrimination is the use of a "molecular beacon", whereby the hybridization probe (molecular beacon) has 3' and 5' reporter and quencher molecules and 3' and 5' sequences which are complementary such that absent an adequate binding target for the intervening sequence the probe will form a hairpin loop. The hairpin loop keeps the reporter and quencher in close proximity resulting in quenching of the fluorophor (reporter) which reduces fluorescence emissions. However, when the molecular beacon hybridizes to the target the fluorophor and the quencher are sufficiently separated to allow fluorescence to be emitted from the fluorophor.

Similarly, primer extension reactions (i.e. mini sequencing, nucleotide-specific extensions, or simple PCR amplification) are useful in sequence discrimination reactions. For example, in mini sequencing a primer anneals to its target DNA immediately upstream of the SNP and is extended with a single nucleotide complementary to the polymorphic site. Where the nucleotide is not complementary, no extension occurs.

Oligonucleotide ligation assays require two sequence-specific probes and one common ligation probe per SNP. The common ligation probe hybridizes adjacent to a sequence-specific probe and when there is a perfect match of the appropriate sequence-specific probe, the ligase joins both the sequence-specific and the common probes. Where there is not a perfect match the ligase is unable to join the sequence-specific and common probes.

Alternatively, an invasive cleavage method requires an oligonucleotide called an Invader™ probe and sequence-specific probes to anneal to the target DNA with an overlap of one nucleotide. When the sequence-specific probe is complementary to the polymorphic base, overlaps of the 3' end of the invader oligonucleotide form a structure that is recognized and cleaved by a Flap endonuclease releasing the 5' arm of the allele specific probe.

5' exonuclease activity or TaqMan™ assay (Applied Biosystems) is based on the 5' nuclease activity of Taq polymerase that displaces and cleaves the oligonucleotide probes hybridized to the target DNA generating a fluorescent signal. It is necessary to have two probes that differ at the polymorphic site wherein one probe is complementary to the 'normal' sequence and the other to the mutation of interest. These probes have different fluorescent dyes attached to the 5' end and a quencher attached to the 3' end when the probes are intact the quencher interacts with the fluorophor by fluorescence resonance energy transfer (FRET) to quench the fluorescence of the probe. During the PCR annealing step the hybridization probes hybridize to target DNA. In the extension step the 5' fluorescent dye is cleaved by the 5' nuclease activity of Taq polymerase, leading to an increase in fluorescence of the reporter dye. Mismatched probes are displaced without fragmentation. The presence of a mutation in a sample is determined by measuring the signal intensity of the two different dyes.

It will be appreciated that numerous other methods for sequence discrimination and detection are known in the art and some of which are described in further detail below. It will also be appreciated that reactions such as arrayed primer extension mini sequencing, tag microarrays and sequence-specific extension could be performed on a microarray. One such array based genotyping platform is the microsphere based tag-it high throughput genotyping array (BORTOLIN S. et al. Clinical Chemistry (2004) 50(11): 2028-36). This method amplifies genomic DNA by PCR followed by sequence-specific primer extension with universally tagged genotyping primers. The products are then sorted on a Tag-It array and detected using the Luminex xMAP system.

Mutation detection methods may include but are not limited to the following:
Restriction Fragment Length Polymorphism (RFLP) strategy - An RFLP gel-based analysis can be used to indicate the presence or absence of a specific mutation at polymorphic sites within a gene. Briefly, a short segment of DNA (typically several hundred base pairs) is amplified by PCR. Where possible, a specific restriction endonuclease is chosen that cuts the short DNA segment when one polymorphism is present but does not cut the short DNA segment when the polymorphism is not present, or vice versa. After incubation of the PCR amplified DNA with this restriction endonuclease, the reaction products are then separated using gel electrophoresis. Thus, when the gel is examined the appearance of two lower molecular weight bands (lower molecular weight molecules travel farther down the gel during electrophoresis) indicates that the DNA sample had a polymorphism was present that permitted cleavage by the specific restriction endonuclease. In contrast, if only one higher molecular weight band is observed (at the molecular weight of the PCR product) then the initial DNA sample has the polymorphism that could not be cleaved by the chosen restriction endonuclease. Finally, if both the higher molecular weight band and the two lower molecular weight bands are visible then the DNA sample contained both polymorphisms, and therefore the DNA sample, and by extension the subject providing the DNA sample, was heterozygous for this polymorphism;

Sequencing - For example the Maxam-Gilbert technique for sequencing (MAXAM AM. and GILBERT W. Proc. Natl. Acad. Sci. USA (1977) 74(4):560-564) involves the specific chemical cleavage of terminally labelled DNA. In this technique four samples of the same labeled DNA are each subjected to a different chemical reaction to effect preferential cleavage of the DNA molecule at one or two nucleotides of a specific base identity. The conditions are adjusted to obtain only partial cleavage, DNA fragments are thus generated in each sample whose lengths are dependent upon the position within the DNA base sequence of the nucleotide(s) which are subject to such cleavage. After partial cleavage is performed, each sample contains DNA fragments of different lengths, each of which ends with the same one or two of the four nucleotides. In particular, in one sample each fragment ends with a C, in another sample each fragment ends with a C or a T, in a third sample each ends with a G, and in a fourth sample each ends with an A or a G. When the products of these four reactions are resolved by size, by electrophoresis on a polyacrylamide gel, the DNA sequence can be read from the pattern of radioactive bands. This technique permits the sequencing of at least 100 bases from the point of labeling. Another method is the dideoxy method of sequencing was published by SANGER et al. (Proc. Natl. Acad. Sci. USA (1977) 74(12):5463-5467). The Sanger method relies on enzymatic activity of a DNA polymerase to synthesize sequence-dependent fragments of various lengths. The lengths of the fragments are determined by the random incorporation of dideoxynucleotide base-specific terminators. These fragments can then be separated in a gel as in the Maxam-Gilbert procedure, visualized, and the sequence determined. Numerous improvements have been made to refine the above methods and to automate the sequencing procedures. Similarly, RNA sequencing methods are also know. For example, reverse transcriptase with dideoxynucleotides have been used to sequence encephalomyocarditis virus RNA (ZIMMERN D. and KAESBERG P. Proc. Natl. Acad. Sci. USA (1978) 75(9):4257-4261). MILLS DR. and KRAMER FR. (Proc. Natl. Acad. Sci. USA (1979) 76(5):2232-2235) describe the use of Qβ replicase and the nucleotide analog inosine for sequencing RNA in a chain-termination mechanism. Direct chemical methods for sequencing RNA are also known (PEATTIE DA. Proc. Natl. Acad. Sci. USA (1979) 76(4):1760-1764). Other methods include those of Donis-Keller et al. (1977, Nucl. Acids Res. 4:2527-2538), SIMONCSITS A. et al. (Nature (1977) 269(5631):833-836), AXELROD VD. et al. (Nucl. Acids Res.(1978) 5(10):3549-3563), and KRAMER FR. and MILLS DR. (Proc. Natl. Acad. Sci. USA (1978) 75(11):5334-5338). Nucleic acid sequences can also be read by stimulating the natural fluoresce of a cleaved nucleotide with a laser while the single nucleotide is contained in a fluorescence enhancing matrix (U.S. Pat. # 5,674,743); In a mini sequencing reaction, a primer that anneals to target DNA adjacent to a SNP is extended by DNA polymerase with a single nucleotide that is complementary to the polymorphic site. This method is based on the high accuracy of nucleotide incorporation by DNA polymerases. There are different technologies for analyzing the primer extension products. For example, the use of labeled or unlabeled nucleotides, ddNTP combined with dNTP or only ddNTP in the mini sequencing reaction depends on the method chosen for detecting the products;

Probes used in hybridization can include double-stranded DNA, single-stranded DNA and RNA oligonucleotides, and peptide nucleic acids. Hybridization methods for the identification of single nucleotide polymorphisms or other mutations involving a few nucleotides are described in the U.S. Pat. 6,270,961; 6,025,136; and 6,872,530. Suitable hybridization probes for use in accordance with the invention include oligonucleotides and PNAs from about 10 to about 400 nucleotides, alternatively from about 20 to about 200 nucleotides, or from about 30 to about 100 nucleotides in length.

A template-directed dye-terminator incorporation with fluorescent polarization-detection (TDI-FP) method is described by FREEMAN BD. et al. (J Mol Diagnostics (2002) 4(4):209-215) for large scale screening;

Oligonucleotide ligation assay (OLA) is based on ligation of probe and detector oligonucleotides annealed to a polymerase chain reaction amplicon strand with detection by an enzyme immunoassay (VILLAHERMOSA ML. J Hum Virol (2001) 4(5):238-48; ROMPPANEN EL. Scand J Clin Lab Invest (2001) 61(2):123-9; IANNONE MA. et al. Cytometry (2000) 39(2):131-40);

Ligation-Rolling Circle Amplification (L-RCA) has also been successfully used for genotyping single nucleotide polymorphisms as described in QI X. et al. Nucleic Acids Res (2001) 29(22):E116;

5' nuclease assay has also been successfully used for genotyping single nucleotide polymorphisms (AYDIN A. et al. Biotechniques (2001) (4):920-2, 924, 926-8.);

Polymerase proofreading methods are used to determine SNPs identities, as described in WO 0181631;

Detection of single base pair DNA mutations by enzyme-amplified electronic transduction is described in PATOLSKY F et al. Nat Biotech. (2001) 19(3):253-257;

Gene chip technologies are also known for single nucleotide polymorphism discrimination whereby numerous polymorphisms may be tested for simultaneously on a single array (EP 1120646 and GILLES PN. et al. Nat. Biotechnology (1999) 17(4):365-70);

Matrix assisted laser desorption ionization time of flight (MALDI-TOF) mass spectroscopy is also useful in the genotyping single nucleotide polymorphisms through the analysis of microsequencing products (HAFF LA. and SMIRNOV IP. Nucleic Acids Res. (1997) 25(18):3749-50; HAFF LA. and SMIRNOV IP. Genome Res. (1997) 7:378-388; SUN X. et al. Nucleic Acids Res. (2000) 28 e68; BRAUN A. et al. Clin. Chem. (1997) 43:1151-1158; LITTLE DP. et al. Eur. J. Clin. Chem. Clin. Biochem. (1997) 35:545-548; FEI Z. et al. Nucleic Acids Res. (2000) 26:2827-2828; and BLONDAL T. et al. Nucleic Acids Res. (2003) 31(24):e155).

Sequence-specific PCR methods have also been successfully used for genotyping single nucleotide polymorphisms (HAWKINS JR. et al. Hum Mutat (2002) 19(5):543-553). Alternatively, a Single-Stranded Conformational Polymorphism (SSCP) assay or a Cleavase Fragment Length Polymorphism (CFLP) assay may be used to detect mutations as described herein.

Provided herein are methods of detecting mutations, which result in an altered levels of SPARC polypeptide. "Altered levels" as used herein include, e.g., a change of about 10%, a change of about 20%, a change of about 30%, a change of about 40%, a change of about 50%, a change of about 75%, and a change of about 100% as compared to wild type levels for the same cell or tissue type. "Altered levels" as used herein also include, e.g., an at least about 2 fold, an at least about 5 fold, an at least about 10 fold, an at least about 20 fold, an at least about 50 fold, an at least about 100 fold, an at least about 200 fold, an at least about 500 fold, and an at least about 1,000 fold change in the level as compared to wild type levels for the same cell or tissue type. Accordingly, included is a method of detecting a mutation in a SPARC sequence occurring in an animal, wherein the mutation is in the promoter of the gene and results in an altered intracellular and/or extracellular levels of a polypeptide encoded by the SPARC gene. The method in accordance with the invention further provides for the detection of mutations wherein the mutation is a point mutation, deletion, insertion or frameshift mutation or combinations thereof. In addition, mutations detectable with the methods described include, e.g., deletion and/or insertions of 1 nucleotide, from 1 to about 5 nucleotides, from about 5 to about 10 nucleotides, from about 10 to about 20 nucleotides, from about 20 to about 50 nucleotides, and from about 50 to about 100 nucleotides, from about 100 nucleotides to about 1000 nucleotides. Altered levels as used herein may be a reduction in the intracellular and/or extracellular SPARC polypeptide.

Mutation detection in accordance with the methods described herein may include thy determination of SPARC promoter sequence of at least about 1 nucleotide, alternatively at least about 5 nucleotides, at least about 10 nucleotides, at least about 50 nucleotides, at least about 100 nucleotides, at least about 200 nucleotides, at least about 300 nucleotides, at least about 400 nucleotides, at least about 500 nucleotides, at least about 1,000 nucleotides, or at least about 2,000 nucleotides.

Provided herein are nucleic acid molecules with one or more SPARC gene promoter mutations, further including a reporter sequence and/or further including a vector. Suitable nucleic acid molecules may include molecules of at least about 10 base pairs, at least about 50 base pairs, at least about 100 base pairs, at least about 200 base pairs, at least about 300 base pairs, at least about 500 base pairs, and at least about 1,000 base pairs. In addition, there is provided a cell including the nucleic acid molecule with a mutant SPARC promoter genotype.

Alternatively, if a subject's genetic sequence data is already known, then obtaining may involve retrieval of the subject's nucleic acid sequence data from a database, followed by determining or detecting the identity of a nucleic acid or genotype at a polymorphism site by reading the subject's nucleic acid sequence at the polymorphic site.

Once the identity of a polymorphism(s) or other mutation is determined or detected an indication may be obtained as to resistance of a subject's cancer to a therapeutic regimen based on the based on the presence or absence of one or more mutations in the subject's SPARC promoter sequence. As described herein, polymorphisms or other mutations in the SPARC promoter sequence, may used to obtain a prognosis or to make a determination regarding the ability of the subject to have a favourable outcome following a chemotherapy, radiotherapy or combination therapy regiment for a cancer. Specifically, to determine whether the subject's cancer is likely to be sensitive to a particular therapeutic regimen or whether the cancer is likely to be resistant to a particular therapeutic regimen. Alternatively, polymorphisms or other mutations in the SPARC promoter may be used to screen subjects to determine the ability of a subject to be treated by a chemotherapy, radiotherapy or combination therapy regimen for cancer. Examples may include subjects to be included in a clinical trial or other similar study. Alternatively, mutations may be used as an indication of a subject's ability to respond to a chemotherapy, radiotherapy or combination therapy regimen directed at the subject's cancer. A mutation may be indicative of a subject's cancer being resistant to a chemotherapy, radiotherapy or combination therapy regimen. Accordingly, a decision regarding the subject's chemotherapy, radiotherapy or combination therapy regimen may be made based on the subject's SPARC gene sequence.

Once a subject's SPARC promoter sequence or SPARC mutational status is determined, such information may be of interest to physicians and surgeons to assist in deciding between potential treatment options, to help determine the degree to which subjects are monitored and the frequency with which such monitoring occurs. Ultimately, treatment decisions may be made in response to factors, both specific to the subject and based on the experience of the physician or surgeon responsible for a subject's care. For example, a subject who has one or more SPARC promoter mutations that are indicative of resistance to a particular therapeutic regimen, may be offered an "alternative therapeutic regimen". Accordingly, a subject who is receiving a first line chemotherapeutic regimen (or is about to receive such a regimen) and has one or more SPARC promoter mutations in their cancer may be preferentially administered an alternative therapeutic regimen. For example, SPARC polypeptide (as described in WO 2004/064785), receptor tyrosine kinase inhibitors (for example Iressa™ (gefitinib), Tarceva™ (erlotinib), Erbitux™ (cetuximab), imatinib mesilate (Gleeve) or Avastin™ (bevacizumab), proteosome inhibitors (for example bortezomib, Velcade); aurora kinase inhibitors (for example ZM447439); rapamycin inhibitors (for example sirolimus, Rapamune); farnesyltransferase inhibitors (for example tipifarnib, Zarnestra); matrix metalloproteinase inhibitors (for example BAY 12-9566; sulfated polysaccharide tecogalan); angiogenesis inhibitors (for example Avastin™ (bevacizumab); TNP-4, a synthetic analogue of fumagillin; carboxyaminotriazole; BB-94 and BB-2516; thalidomide; interleukin-12; linomide; peptide fragments; and antibodies to vascular growth factors and vascular growth factor receptors); cyclo-oxygenase inhibitors, platelet derived growth factor receptor inhibitors, protein kinase C inhibitors, mitogen-activated protein kinase kinase inhibitors, histonedeacetylase inhibitors, small hypoxia-inducible factor inhibitors, hedgehog inhibitors, and TGF-β signalling inhibitors. Furthermore, "Immunotherapeutic agent", as used herein, would also be considered an alternative therapeutic regimen. For example, agents used to transfer the immunity of an immune donor, e.g., another person or an animal, to a host by inoculation. The term embraces the use of serum or gamma globulin containing performed antibodies produced by another individual or an animal; nonspecific systemic stimulation; adjuvants; active specific immunotherapy; and adoptive immunotherapy. Adoptive immunotherapy refers to the treatment of a disease by therapy or agents that include host inoculation of sensitized lymphocytes, transfer factor, immune RNA, or antibodies in serum or gamma globulin. Such alternative therapeutics may be administered alone or in combination or in combination with other therapeutic regimens described herein.

A subject may be monitored on an ongoing basis for SPARC promoter mutations during the course of chemotherapy, radiotherapy or combination therapy regimen. A response to a selected regimen may be predicted by the presence or absence of one or more SPARC promoter mutations as described herein. An improved response may include an increased likelihood of survival, reduction in tumor size or reduction in the rate of metastasis. Tumor staging provides a method to assess the size and spread, and by extension guide prognosis of a tumor in a subject. The TNM tumor staging system uses three components to express the anatomic extent of disease: T is a measure of the local extend of tumor spread (size), N indicates the presence or absence of metastatic spread to regional lymph nodes, and M specifies the presence or absence of metatstatic spread to distant sites. The combination of these three classifications combine to provide a stage grouping. Clinical TNM (cTNM) defines the tumor based on clinical evidence. Pathologic TNM (pTNM) defines the tumor based on examination of a surgically resected specimen.

Changes in tumor size may be observed by various imaging methods known to physicians or surgeons in the field of oncology therapy and diagnostics. Examples of imaging methods include positron emission tomography (PET) scanning, computed tomography (CT) scanning, PET/CT scanning, magnetic resonance imaging (MRI), chemical shift imaging, radiography, bone-scan, mammography, fiberoptic colonoscopy or ultrasound. Contrast agents, tracers and other specialized techniques may also be employed to image specific types of cancers, or for particular organs or tissues, and will be known to those skilled in the art. Changes in rate of metastasis may also be observed by the various imaging methods, considering particularly the appearance, or frequency of appearance, of tumors distal to the primary site. Alternatively, the presence of tumor cells in lymph nodes adjacent and distal to the primary tumor site may also be detected and used to monitor metastasis.

### 2.B. Methods and Materials

### Cell Culture and Maintenance

A human colorectal cancer cell line, MIP-101, and uterine sarcoma cell line, MES-SA were maintained in Dulbecco's modified Eagle's medium (DMEM) supplemented with 10% fetal bovine serum (FBS), 1% penicillin/streptomycin (Invitrogen, Carlsbad, CA) at 37°C and 5% CO₂. MIP/5FU (MIP-101 cells resistant to 5-fluorouracil), MIP/CIS (MIP-101 cells resistant to cisplatin) and MIP/CPT (MIP-101 cells resistant to irinotecan) were maintained as described above, but with the inclusion of the respective resistant drug (5-flurouracil 200 µM, cisplatin 10 µM, irinotecan 20 µM). MES-SA cells resistant to doxorubicin (MES-SA/DX5) were acquired from ATCC (# CRL-1977) and maintained according to supplier's recommendations.

### Development of Resistant Cell Lines

Resistant MIP101 cells were developed following long-term incubation with incremental concentrations of chemotherapeutic agents 5-fluorouracil (5FU; 10-500 µM) (Sigma, St. Louis, MO), cisplatin (CIS; 1-100 µM) (Sigma, St. Louis, MO) or irinotecan (CPT; 1-50 µM) (Pharmacia, Kalamazoo, MI) over 3 months. The concentration of each chemotherapeutic agent was increased by 10% every 2 weeks for a 6 month period.

### Isolation of Cellular DNA

10-20 million colorectal cancer cells from sensitive (MIP101, MES-SA) and resistant (MIP/5FU, MIP/CPT, MIP/CIS, MES-SA/DX5) cells were collected and DNA extracted based on the manufacturer's protocol (DNA Extraction Kit, Gentra Systems, Inc.).

### Sequencing Reactions

Overlapping sections of the SPARC promoter were sequenced using the BigDye™ Terminator v 3.1 Cycle Sequencing Kit (BD Biosciences) following manufacturer's instructions. Briefly, 4 µl of reaction premix, 2 µl of sequencing buffer, 3.2 pmol of primer and 50-100 ng of isolated cellular DNA was combined with water to make a final volume of 20 µl. Cycle sequencing was performed using the following parameters: an initial denaturation step at 95°C for 2 minutes, followed by 30 cycles of 95°C for 30 seconds, 60°C for 30 seconds, 72°C for 1 minute, with a final extension at 72°C for 10 min. Sequencing reaction amplicons were purified by incubation with AMPure magnetic beads (Agencourt Bioscience Corporation), following manufacturer's instructions. Briefly, the sequencing reaction mixture was incubated with AMPure magnetic beads for 10 min, followed by collection of beads with a magnetic plate. The collected beads were washed once with 70% ethanol, and the PCR amplicons eluted with Tris-EDTA, ph 8.0. The eluted sequencing reaction amplicons were sequenced using an ABI Prism 3700 DNA analyzer. Sequence chromatograms generated by ABI Prism 3700 DNA Analyzer were analyzed and assembled by Phred-Phrap programs and assembled by Consed programs sing reference sequences from UCSC Bioinformatics for the identification of mutations.

### Analytical RT-PCR.

To determine SPARC mRNA levels Analytical RT-PCR could be performed as described by TAI, IT. et al. (J. Clin Invest. (2005) 115:1493-1502). Briefly, total RNA would be extracted from cultured cells, at 75% confluence and under similar culture conditions, using TRIZOL reagent, following manufacturer's instructions. RT-PCR could be performed using One-Step RT-PCR (BD Biosciences), following manufacturer's instructions. SPARC-specific primers such as SEQ ID NO: 52 and SEQ ID NO: 53. GAPDH-specific primers SEQ ID NO: 54 and SEQ ID NO: 55 could be used. The reaction conditions for such a reaction would be 50°C for 1 hour, followed by 45 cycles at 94°C for 1 minute, 65°C for 1 minute, 72°C for 2 minutes, and 72°C for 10 minutes. PCR products could then be separated on 1% agarose gel electrophoresis.

### Protein Extracts and Immunoblots

To determine SPARC protein levels, extracts and immunoblots could be prepared as described by TAI IT *et al.* 2005, supra. Briefly, total protein could be extracted from cell lysates using CHAPS lysis buffer (50 mM PIPES/HCl, pH 6.5; 2 mM EDTA; 0.1% CHAPS; 20 µg/ml Leupeptin; 10 µg/ml Pepstatin A; 10 µg/ml Aprotinin; 5 mM DTT; 1 mM PMSF). The proteins could be resolved by SDS-PAGE (10% gel) under reducing conditions and electrotransferred onto a PVDF membrane and analyzed by Western blot. Antibodies to SPARC (1 µg/ml; Haematologic Technologies Inc.) and alpha-tubulin (0.2 µg/ml; Sigma-Aldrich) could be used.

SPARC mRNA and protein levels have been reported to be low in cell lines resistant to chemotherapy. TAI IT. *et al.* 2005, *supra* describe decreased SPARC mRNA and protein levels in MIP resistant cells (MIP/5FU, MIP/CPT, MIP/CIS and MES-SA/DX5, among others), as evaluated by RT-PCR and western blotting. Significant reductions in SPARC-specific mRNA compared with the sensitive parental lines was observed for all cell lines tested. This result was of particular interest as it was consistent over both colorectal carcinoma and uterine sarcoma cells, resistant to four different chemotherapeutic agents. Protein levels of SPARC were also significantly decreased in the resistant cell lines, again reflected in both colorectal carcinoma and uterine sarcoma cell lines, resistant to flour different chemotherapeutic agents.

### 2. EXAMPLES

### Example 1

### Mutations in SPARC regulatory regions in MIP/5FU cell lines

A region spanning 2963 base pairs spanning a regulatory region of SPARC∼1400 bp upstream of exon 1, exon 1 and ∼1300 of intron 1 (SEQ ID NO: 9) was sequenced on both strands using primers corresponding to SEQ ID NOs: 34 to 39 in both sensitive (MIP-101) and 5FU resistant (MIP/5FU) cell lines. Several mutations were identified in the regulatory region between nucleotides 1 to 1370 of SEQ ID NO: 1 that were not found in the sensitive cell line. The individual mutations are listed in TABLE 1- mutations specifically identified to date in the MIP/SFCT cell lines are shown in TABLE 5 *infra*.

### Example 2

### Mutations in SPARC regulatory regions in MIP/CPT cell lines

A region spanning 2963 base pairs spanning a regulatory region of SPARC∼1400 bp upstream of exon 1, exon 1 and ∼1300 of intron 1 (SEQ ID NO: 9) was sequenced on both strands using primers corresponding to SEQ ID NOs: 34-39 in both sensitive (MIP-101) and 5FU resistant (MIP/5FU) cell lines. Several mutations were identified in the regulatory region between nucleotides 1 to 1370 of SEQ ID NO: 1 that were not found in the sensitive cell line. The individual mutations are listed in TABLE 1- mutations specifically identified to date in the MIP/CPT cell lines are shown in TABLE 5 *infra*.

### Example 3

### Mutations in SPARC regulatory regions in MIP/CIS cell lines

A region spanning 2963 base pairs spanning a regulatory region of SPARC∼1400 bp upstream of exon 1, exon 1 and ∼1300 of intron 1 (SEQ ID NO: 9) was sequenced on both strands using primers corresponding to SEQ ID NOs: 34-39 in both sensitive (MIP-101) and 5FU resistant (MIP/5FU) cell lines. Several mutations were identified in the regulatory region between nucleotides 1 to 1370 of SEQ ID NO: 1 that were not found in the sensitive cell line. The individual mutations are listed in TABLE 1- mutations specifically identified to date in the MIP/CIS cell lines are shown in TABLE 5 *infra*.

### Example 4

### Mutations in SPARC regulatory regions in MES-SA/DX5 cell lines

A region spanning 2963 base pairs spanning a regulatory region of SPARC ∼1400 bp upstream of exon 1, exon 1 and ∼1300 of intron 1 (SEQ ID NO: 9) was sequenced on both strands using primers corresponding to SEQ ID NOs: 34-39 in both sensitive (MIP-101) and 5FU resistant (MIP/5FU) cell lines. Several mutations were identified in the regulatory region between nucleotides 1 to 1370 of SEQ ID NO: 1 that were not found in the sensitive cell line. The individual mutations are listed in TABLE 1- mutations specifically identified to date in the MES-SA/DX5 cell lines are shown in TABLE 5 *infra*. The results shown in TABLE 5 below represent mutations identified to date in various drug resistant cell lines and the absence of a mutation (i.e. no √) is not meant to represent that such a mutation is not possible or even likely, only that to date not such mutation has been identified in a given resistant cell line with a given chemotherapeutic regimen.

**TABLE 5**

| | | **Resistant Cell Lines** **(MIP = colorectal cancer, MES - uterine sarcoma)** **Cell Line/Therapeutic Regimen** | | | |
|---|---|---|---|---|---|
| **Mutation Identifier** | **SPARC MUTATIONS RELATIVE TO SEQ ID NO:1** | **MIP/ 5FU** | **MIP/ CPT** | **MIP/ cisplatin** | **MES/ doxorubicin** |
| (a) | 295delG and 301A>CC | | | √ | |
| (b) | 315insA and 316G>A | | | √ | |
| (c) | 341C>T, 342T>C, 344A>C, 347C>A, 354G>A, 356G>A, 359T>G and 363G>A | | | √ | |
| (d) | 395C>T and 396A>T | | √ | √ | √ |
| (e) | 488T>C | | √ | | |
| (f) | 533A>G | | | | √ |
| (g) | 734G>A | √ | √ | √ | √ |
| (h) | 734delG | √ | √ | √ | √ |
| (i) | 769T>A, 771G>C and 774G>C | | √ | | |
| (j) | 784T>C, 786T>G and 788T>A | | √ | | |
| (k) | 793T>G. 796T>A and 800T>C | | | | |
| (l) | 820C>A and 824T>A | | √ | | |
| (m) | 864T>A and 867T>A | | √ | | |
| (n) | 895T>G, 899T>A, 901G>A and 904G>A | | √ | √ | √ |
| (o) | 901G>A | √ | √ | √ | √ |
| (p) | 901G>A, 904G>A, 929A>G and 934delC | √ | √ | √ | |
| (q) | 922T>G, 928T>G and 935T>G | | √ | | |
| (r) | 982T>C, 984T>G, 985G>A, 991G>A, 995G>A, 999C>T and 1000T>G | | √ | | |
| (s) | 1063G>C | √ | √ | √ | |
| (t) | 1079G>A | √ | √ | √ | |
| (u) | 1085delC, 1086delA, 1087delG, 1089G>A, 1090T>C, 1091T>C and 1092G>T | √ | √ | √ | |
| (v) | 1161C>A, 1162T>C, 1163delA and 1164delT | √ | √ | √ | |
| (w) | 1215C>T, 1216delG, 1217delG, 1218delG, 1219delG, 1220delT, 1221delG, 1222delG, 1223delA, 1224delG, 1225delG, 1226delG, 1227delG, 1228delA, 1229delG, 1230delA, 1231delT, 1232delA, 1233delG, 1234delA, 1235delC, 1236delC and 1237C>T | √ | √ | √ | |

## Claims

1. A method for predicting responsiveness of a subject to a therapeutic regimen, the subject having, or suspected of having a cancer, the method comprising determining a presence or an absence of one or more a secreted protein, acidic, cysteine-rich (SPARC) promoter mutations in the subject's cancer, wherein said presence or absence of one or more SPARC promoter mutations is indicative of a sensitivity of the subject's cancer to the therapeutic regimen, wherein the one or more SPARC promoter mutations, are selected from the following mutations relative to SEQ will NO: 1:
(a) 295delG and 301A>CC;
(b) 315insA and 316G>A;
(c) 341C>T, 342T>C, 344A>C, 347C>A, 354G>A, 356G>A, 359T>G and 363G>A;
(d) 395C>T and 396A>T;
(e) 488T>C;
(f) 533A>G;
(g) 734G>A;
(h) 734delG;
(i) 769T>A, 771 G>C and 774G>C;
(j) 784T>C, 786T>G and 788T>A;
(k) 793T>G, 796T>A and 800T>C;
(l) 820C>A and 824T>A;
(m) 864T>A and 867T>A;
(n) 895T>G, 899T>A, 901G>A and 904G>A;
(o) 901G>A;
(p) 901G>A, 904G>A, 929A>G and 934delC;
(q) 922T>G, 928T>G and 93 5T>G;
(r) 982T>C, 984T>G, 985G>A, 991G>A, 995G>A, 999C>T and 1000T>G;
(s) 1063G>C;
(t) 1079G>A;
(u) 1085delC, 1086delA, 1087delG, 1089G>A, 1090T>C, 1091T>C and 1092G>T;
(v) 1161C>A, 1162T>C, 1163delA and 1164delT; and
(w) 1215C>T, 1216delG, 1217delG, 1218delG, 1219delG, 1220delT, 1221delG, 1222delG, 1223delA, 1224delG, 1225delG, 1226delG, 1227delG, 1228delA, 1229delG, 1230delA, 1231delT, 1232delA, 1233delG, 1234delA, 1235delC, 1236delC and 1237C>T, and
wherein the therapeutic regimen comprises administration of one or more chemotherapeutic agents.

2. The method of claim 1, wherein the promoter mutation results in altered intracellular and/or extracellular levels of a polypeptide encoded by the SPARC promoter sequence.

3. The method of claim 1 or 2, wherein the therapeutic regimen further comprises radiotherapy.

4. The method of claim 1, wherein the chemotherapeutic agent is selected from 5-flurouracil, irinotecan, cisplatin, doxorubicin or combinations thereof.

5. The method of claim 1, wherein the one or more SPARC promoter mutations are indicative of the cancer's resistance to the therapeutic regimen.

6. The method of any one of claims 1-5, further comprising obtaining SPARC promoter information for the subject.

7. The method of any one of claims 1-6, wherein the one or more SPARC promoter mutations are determined using a nucleic acid sample from the subject.

8. The method of claim 7, wherein the nucleic acid sample has been obtained from the subject.

9. The method of claim 8, wherein the nucleic acid sample is from a biological sample taken from the subject's cancer.

10. The method of any one of claims 1-9, wherein said determining the presence or an absence of one or more SPARC promoter mutations in the subject's cancer is done using one or more of the following techniques:
(a) restriction fragment length analysis;
(b) sequencing;
(c) micro-sequencing assay;
(d) hybridization;
(e) invader assay;
(f) gene chip hybridization assays;
(g) oligonucleotide ligation assay;
(h) ligation rolling circle amplification;
(i) 5' nuclease assay;
(j) polymerase proofreading methods;
(k) allele specific PCR;
(l) matrix assisted laser desorption ionization time of flight (MALDI-TOF) mass spectroscopy;
(m) ligase chain reaction assay;
(n) enzyme-amplified electronic transduction;
(o) single base pair extension assay; and
(p) reading sequence data.

11. The method of any one of claims 1-10, wherein the cancer is selected from the following: carcinoma; melanoma; sarcoma; lymphoma; and leukaemia.

12. The method of claim 11, wherein the carcinoma is selected from the following: squamous cell; adeno; small cell; large cell carcinoma and combinations thereof.

13. The method of claim 12, wherein the carcinoma is derived from a primary tumor located in nasopharygeal tissue, head and neck, lung, esophagus, stomach, intestine, rectum, kidney, urinary tract, prostate, testes, ovary, uterus, cervix, vagina, skin, or endocrine glands.

14. The method of claim 11, wherein the sarcoma is selected from the following: muscle; adipose; fibrous; vascular; and neural sarcoma.

15. The method of claim 11, wherein the lymphoma is selected from the following: Hodgkin's Disease; and Non-Hodgkins Lymphoma.

16. The method of claim 11, wherein the leukaemia is selected from the following: lymphoid; and myeloid leukaemia.

17. The method of claim 11, wherein the cancer is a colorectal carcinoma, prostate carcinoma, ovarian carcinoma or osteosarcoma.

18. The method of any one of claims 1-17, wherein the cancer is a SPARC inhibited cancer.

19. The method of claim 18, wherein the SPARC inhibited cancer is selected from colorectal carcinoma, prostate carcinoma, ovarian carcinoma, osteosarcoma and uterine sarcoma.

20. A method for selecting a group of subjects for determining the efficacy of a therapeutic regimen known or suspected of being useful for the treatment of cancer, the method comprising determining a genotype at one or more mutation sites in a SPARC promoter for each subject, wherein said genotype is indicative of the subject's sensitivity to a therapeutic regimen and wherein the one or more SPARC promoter mutations, are selected from the following mutations relative to SEQ ID NO: 1:
(a) 295delG and 301A>CC;
(b) 315insA and 316G>A;
(c) 341C>T, 342T>C, 344A>C, 347C>A, 354G>A, 356G>A, 359T>G and 363G>A;
(d) 395C>T and 396A>T;
(e) 488T>C;
(f) 533A>G;
(g) 734G>A;
(h) 734delG;
(i) 769T>A, 771 G>C and 774G>C;
(j) 784T>C, 786T>G and 788T>A;
(k) 793T>G, 796T>A and 800T>C;
(l) 820C>A and 824T>A;
(m) 864T>A and 867T>A;
(n) 895T>G, 899T>A, 901G>A and 904G>A;
(o) 901G>A;
(p) 901G>A, 904G>A, 929A>G and 934delC;
(q) 922T>G, 928T>G and 935T>G;
(r) 982T>C, 984T>G, 985G>A, 991G>A, 995G>A, 999C>T and 1000T>G;
(s) 1063G>C;
(t) 1079G>A;
(u) 1085delC, 1086delA, 1087delG, 1089G>A, 1090T>C, 1091T>C and 1092G>T;
(v) 1161C>A, 1162T>C, 1163delta and 1164delT; and
(w) 1215C>T, 1216delG, 1217delG, 1218delG, 1219delG, 1220delT, 1221delG, 1222delG, 1223delA, 1224delG, 1225delG, 1226delG, 1227delG, 1228delA, 1229delG, 1230delA, 1231delT, 1232delA, 1233delG, 1234delA, 1235delC, 1236delC and 1237C>T, and
wherein the therapeutic regimen is a chemotherapy.

21. The method of claim 20, further comprising determining each subject's sensitivity to the therapeutic regimen and comparing subject response to the therapeutic regimen based on the genotype of the subject.

22. The method of claim 20, wherein the chemotherapy is 5-flurouracil, irinotecan, cisplatin, doxorubicin or combinations thereof.

23. A method of identifying a subject with an increased sensitivity to a chemotherapy for treating a cancer in a subject in need thereof, comprising the step of screening a population of subjects to identify those subjects that have a mutation in their SPARC promoter sequence, wherein the identification of a subject with a mutation is predictive of decreased responsiveness to the chemotherapy, wherein the SPARC promoter mutation, is selected from one or more of the following mutations relative to SEQ ID NO: 1:
(a) 295delG and 301 A>CC;
(b) 315insA and 316G>A;
(c) 341C>T, 342T>C, 344A>C, 347C>A, 354G>A, 356G>A, 359T>G and 363G>A;
(d) 395C>T and 396A>T;
(e) 488T>C;
(f) 533A>G;
(g) 734G>A;
(h) 734delG;
(i) 769T>A, 771 G>C and 774G>C;
(j) 784T>C, 786T>G and 788T>A;
(k) 793T>G, 796T>A and 800T>C;
(l) 820C>A and 824T>A;
(m) 864T>A and 867T>A;
(n) 895T>G, 899T>A, 901 G>A and 904G>A;
(o) 901G>A;
(p) 901G>A, 904G>A, 929A>G and 934delC;
(q) 922T>G, 928T>G and 935T>G;
(r) 982T>C, 984T>G, 985G>A, 991G>A, 995G>A, 999C>T and 1000T>G;
(s) 1063G>C;
(t) 1079G>A;
(u) 1085delC, 1086delA, 1087delG, 1089G>A, 1090T>C, 1091T>C and 1092G>T;
(v) 1161C>A, 1162T>C, 1163delta and 1164delT; and
(w) 1215C>T, 1216delG, 1217delG, 1218delG, 1219delG, 1220delT, 1221delG, 1222delG, 1223delA, 1224delG, 1225delG, 1226delG, 1227delG, 1228delA, 1229delG, 1230delA, 1231delT, 1232delA, 1233delG, 1234delA, 1235delC, 1236delC and 1237C>T.

## Patentansprüche

1. Verfahren zum Vorhersagen des Ansprechens eines Subjekts auf ein Therapieschema, wobei das Subjekt Krebs hat oder im Verdacht steht, Krebs zu haben, und das Verfahren das Feststellen von An- oder Abwesenheit einer oder mehrerer Promotormutationen des sekretierten aziden cysteinreichen Proteins (SPARC) im Krebs des Subjekts umfasst, wobei An- oder Abwesenheit von einer oder mehreren SPARC-Promotor-Mutationen auf eine Sensitivität des Krebses des Subjekts gegenüber dem Therapieschema hinweist, wobei die eine oder die mehreren SPARC-Promotor-Mutationen ausgewählt sind aus den folgenden, relativ zu SEQ. ID Nr. 1 definierten Mutationen:
(a) 295delG und 301A>CC;
(b) 315insA und 316G>A;
(c) 341C>T, 342T>C, 344A>C, 347C>A, 354G>A, 356G>A, 359T>G und 363G>A;
(d) 395C>T und 396A>T;
(e) 488T>C;
(f) 533A>G;
(g) 734G>A;
(h) 734delG;
(i) 769T>A, 771 G>C und 774G>C;
(j) 784T>C, 786T>G und 788T>A;
(k) 793T>G, 796T>A und 800T>C;
(l) 820C>A und 824T>A;
(m) 864T>A und 867T>A;
(n) 895T>G, 899T>A, 901 G>A und 904G>A;
(o) 901 G>A;
(p) 901 G>A, 904G>A, 929A>G und 934delC;
(q) 922T>G, 928T>G und 935T>G;
(r) 982T>C, 984T>G, 985G>A, 991 G>A, 995G>A, 999C>T und 1000T>G;
(s) 1063G>C;
(t) 1079G>A;
(u) 1085delC, 1086delA, 1087delG, 1089G>A, 1090T>C, 1091 T>C und 1092G>T;
(v) 1161C>A, 1162T>C, 1163delA und 1164drlT; und
(w) 1215C>T, 1216delG, 1217delG, 1218delG, 1219delG, 1220delT, 1221delG, 1222delG, 1223delA, 1224delG, 1225delG, 1226delG, 1227delG, 1228delA, 1229delG, 1230delA, 1231delT, 1232delA, 1233delG, 1234delA, 1235delC, 1236delC und 1237C>T, und
wobei das Therapieschema die Verabreichung eines oder mehrerer chemotherapeutischer Agentien umfasst.

2. Verfahren nach Anspruch 1, wobei die Promotormutation zu geänderten intrazellulären und/oder extrazellulären Mengen eines Polypeptids führt, das von der SPARC-Promotor-Sequenz kodiert ist.

3. Verfahren nach Anspruch 1 oder 2, wobei das Therapieschema ferner eine Radiotherapie umfasst.

4. Verfahren nach Anspruch 1, wobei das chemotherapeutische Agens ausgewählt ist aus 5-Fluorouracil, Irinotecan, Cisplatin, Doxorubicin oder Kombinationen davon.

5. Verfahren nach Anspruch 1, wobei die eine oder die mehreren SPARC-Promotor-Mutationen auf die Resistenz des Krebses gegenüber dem Therapieschema hinweisen.

6. Verfahren nach einem der Ansprüche 1 bis 5, ferner umfassend das Beschaffen von SPARC-Promotor-Information für das Subjekt.

7. Verfahren nach einem der Ansprüche 1 bis 6, wobei die eine oder die mehreren SPARC-Promotor-Mutationen unter Verwendung einer Nukleinsäureprobe von dem Subjekt bestimmt werden.

8. Verfahren nach Anspruch 7, wobei die Nukleinsäureprobe dem Subjekt entnommen wurde.

9. Verfahren nach Anspruch 8, wobei die Nukleinsäureprobe aus einer biologischen Probe stammt, die dem Krebs des Subjekts entnommen wurde.

10. Verfahren nach einem der Ansprüche 1 bis 9, wobei das Bestimmen von An- oder Abwesenheit von einer oder mehreren SPARC-Promotor-Mutationen im Krebs des Subjekts mittels eines oder mehrerer der folgenden Techniken durchgeführt wird:
(a) Restriktionsfragmentlängenanalyse;
(b) Sequenzieren;
(c) Mikrosequenzierungstest;
(d) Hybridisierung;
(e) Invadertest;
(f) Gen-Chip-Hybridisierungstest;
(g) Oligonukleotid-Ligationstest;
(h) Ligations-Rolling-Circle-Amplifizierung;
(i) 5'Nukleasetest;
(j) Polymerase-Korrekturleseverfahren;
(k) Allel-spezifische PCR;
(l) matrixunterstützte Laserdesorptions-Ionisations-Flugzeit (MALDI-TOF)-Massenspektrometrie;
(m) Ligase-Kettenreaktionstest;
(n) Enzym-amplifizierte elektronische Transduktion;
(o) Einzelbasenpaar-Verlängerungstest; und
(p) Lesen von Sequenzdaten.

11. Verfahren nach einem der Ansprüche 1 bis 10, wobei der Krebs ausgewählt ist aus den folgenden: Karzinom, Melanom, Sarkom, Lymphom und Leukämie.

12. Verfahren nach Anspruch 11, wobei das Karzinom ausgewählt ist aus den folgenden: Schuppenzell-, Adeno-, Kleinzell-, Großzellkarzinom und Kombinationen davon.

13. Verfahren nach Anspruch 12, wobei das Karzinom von einem Primärtumor abgeleitet ist, der sich in einem nasopharyngealen Gewebe, in Kopf und Nacken, Lunge, Speiseröhre, Magen, Darm, Rektum, Niere, Harntrakt, Prostata, Hoden, Eierstock, Uterus, Zervix, Vagina, Haut oder endokrinen Drüsen befindet.

14. Verfahren nach Anspruch 11, wobei das Sarkom ausgewählt ist aus Muskel-, Fettgewebe-, fibrösem, vaskulärem und neuralem Sarkom.

15. Verfahren nach Anspruch 11, wobei das Lymphom ausgewählt ist aus Hodgkin'scher Krankheit und Non-Hodgkin's-Lymphom.

16. Verfahren nach Anspruch 11, wobei die Leukämie ausgewählt ist aus lymphoider und myeloider Leukämie.

17. Verfahren nach Anspruch 11, wobei der Krebs ein kolorektales Karzinom, Prostatakarzinom, Eierstockkarzinom oder Osteosarkom ist.

18. Verfahren nach einem der Ansprüche 1 bis 17, wobei der Krebs ein SPARC-gehemmter Krebs ist.

19. Verfahren nach Anspruch 18, wobei der SPARC-gehemmte Krebs ausgewählt ist aus kolorektalem Karzinom, Prostatakarzinom, Eierstockkarzinom, Osteosarkom und uterines Sarkom.

20. Verfahren zum Auswählen einer Gruppe von Subjekten für das Bestimmen der Wirksamkeit eines Therapieschemas, von dem bekannt ist oder erwartet wird, dass es für die Behandlung von Krebs geeignet ist, wobei das Verfahren das Bestimmen eines Genotyps an einer oder mehreren Mutationsstellen in einem SPARC-Promotor für jedes Subjekt umfasst, wobei der Genotyp auf die Sensitivität des Subjekts gegenüber einem Therapieschema hinweist und wobei die eine oder die mehreren SPARC-Promotor-Mutationen ausgewählt sind aus den folgenden Mutationen, die im Hinblick auf SEQ. ID Nr. 1 definiert sind:
(a) 295delG und 301A>CC;
(b) 315insA und 316G>A;
(c) 341C>T, 342T>C, 344A>C, 347C>A, 354G>A, 356G>A, 359T>G und 363G>A;
(d) 395C>T und 396A>T;
(e) 488T>C;
(f) 533A>G;
(g) 734G>A;
(h) 734delG;
(i) 769T>A, 771 G>C und 774G>C;
(j) 784T>C, 786T>G und 788T>A;
(k) 793T>G, 796T>A und 800T>C;
(l) 820C>A und 824T>A;
(m) 864T>A und 867T>A;
(n) 895T>G, 899T>A, 901 G>A und 904G>A;
(o) 901 G>A;
(p) 901 G>A, 904G>A, 929A>G und 934delC;
(q) 922T>G, 928T>G und 935T>G;
(r) 982T>C, 984T>G, 985G>A, 991 G>A, 995G>A, 999C>T und 1000T>G;
(s) 1063G>C;
(t) 1079G>A;
(u) 1085delC, 1086delA, 1087delG, 1089G>A, 1090T>C, 1091 T>C und 1092G>T;
(v) 1161C>A, 1162T>C, 1163delA und 1164delT; und
(w) 1215C>T, 1216delG, 1217delG, 1218delG, 1219delG, 1220delT, 1221delG, 1222delG, 1223delA, 1224delG, 1225delG, 1226delG, 1227delG, 1228delA, 1229delG, 1230delA, 1231delT, 1232delA, 1233delG, 1234delA, 1235delC, 1236delC und 1237C>T, und
wobei das Therapieschema eine Chemotherapie ist.

21. Verfahren nach Anspruch 20, ferner umfassend das Bestimmen der Sensitivität jedes Subjekts gegenüber dem Therapieschema und Vergleichen des Ansprechens des Subjekts auf das Therapieschema basierend auf dem Genotyp des Subjekts.

22. Verfahren nach Anspruch 20, wobei die Chemotherapie 5-Fluorouracil, Irinotecan, Cisplatin, Doxorubicin oder Kombinationen davon ist/sind.

23. Verfahren zum Identifizieren eines Subjekts mit einer erhöhten Sensitivität gegenüber einer Chemotherapie zum Behandeln eines Krebses in einem Subjekt, das dessen bedarf, umfassend den Schritt des Screenens einer Population von Subjekten, um jene Subjekte zu identifizieren, die eine Mutation in ihrer SPARC-Promotor-Sequenz haben, wobei die Identifizierung eines Subjekts mit einer Mutation ein reduziertes Ansprechen auf die Chemotherapie vorhersagt, wobei die SPARC-Promotor-Mutation ausgewählt ist aus einer oder mehreren der folgenden Mutationen, die im Hinblick auf SEQ. Nr. 1 definiert sind:
(a) 295delG und 301A>CC;
(b) 315insA und 316G>A;
(c) 341 C>T, 342T>C, 344A>C, 347C>A, 354G>A, 356G>A, 359T>G und 363G>A;
(d) 395C>T und 396A>T;
(e) 488T>C;
(f) 533A>G;
(g) 734G>A;
(h) 734delG;
(i) 769T>A, 771 G>C und 774G>C;
(j) 784T>C, 786T>G und 788T>A;
(k) 793T>G, 796T>A und 800T>C;
(l) 820C>A und 824T>A;
(m) 864T>A und 867T>A;
(n) 895T>G, 899T>A, 901 G>A und 904G>A;
(o) 901 G>A;
(p) 901 G>A, 904G>A, 929A>G und 934delC;
(q) 922T>G, 928T>G und 935T>G;
(r) 982T>C, 984T>G, 985G>A, 991 G>A, 995G>A, 999C>T und 1000T>G;
(s) 1063G>C;
(t) 1079G>A;
(u) 1085delC, 1086delA, 1087delG, 1089G>A, 1090T>C, 1091 T>C und 1092G>T;
(v) 1161C>A, 1162T>C, 1163delA und 1164delT; und
(w) 1215C>T, 1216delG, 1217delG, 1218delG, 1219delG, 1220delT, 1221delG, 1222delG, 1223delA, 1224delG, 1225delG, 1226delG, 1227delG, 1228delA, 1229delG, 1230delA, 1231delT, 1232delA, 1233delG, 1234delA, 1235delC, 1236delC und 1237C>T.

## Revendications

1. Procédé de prédiction de la sensibilité d'un sujet à un régime thérapeutique, le sujet ayant un cancer, ou étant soupçonné d'en avoir un, le procédé comprenant la détermination de la présence ou de l'absence d'une ou plusieurs mutations du promoteur de la protéine sécrétée, acide et riche en cystéine (SPARC) dans le cancer du sujet, dans lequel ladite présence ou absence d'une ou plusieurs mutations du promoteur de SPARC indique la sensibilité du cancer du suj et au régime thérapeutique, dans lequel une ou plusieurs mutations du promoteur de SPARC sont sélectionnées parmi les mutations suivantes relatives à SEQ N : 1 :
(a) 295delG et 301A>CC ;
(b) 315insA est 316G>A ;
(c) 341C>T, 342T>C, 344A>C, 347C>A, 354G>A, 356G>A, 359T>G et 363G>A ;
(d) 395C>T et 396A>T ;
(e) 488T>C;
(f) 533A>G;
(g) 734G>A;
(h) 734delG;
(i) 769T>A, 771G>C et 774G>C ;
(j) 784T>C, 786T>G et 788T>A ;
(k) 793T>G, 796T>A et 800T>C ;
(l) 820C>A et 824T>A ;
(m) 864T>A et 867T>A ;
(n) 895T>G, 899T>A, 901G>A est 904G>A ;
(o) 901G>A;
(p) 901 G>A, 904G>A, 929A>G et 934delC ;
(q) 922T>G, 928T>G et 935T>G ;
(r) 982T>C, 984T>G, 985G>A, 991G>A, 995G>A, 999C>T et 1000T>G ;
(s) 1063G>C ;
(t) 1079G>A ;
(u) 1085delC, 1086delA, 1087delG, 1089G>A, 1090T>C, 1091T>C et 1092G>T,
(v) 1161C>A, 1162T>C, 1163delA et 1164delT ; et
(w) 1215C>T, 1216delG, 1217delG, 1218delG, 1219delG, 1220delT, 1221delG, 1222delG, 1223delA, 1224delG, 1225delG, 1226delG, 1227delG, 1228delA, 1229delG, 1230delA, 1231delT, 1232delA, 1233delG, 1234delA, 1235delC, 1236delC et 1237C>T, et
dans lequel le régime thérapeutique comprend l'administration d'un ou plusieurs agents chimiothérapeutiques.

2. Procédé selon la revendication 1, dans lequel la mutation du promoteur se traduit par des taux intracellulaires et/ou extracellulaires altérés d'un polypeptide codé par la séquence promotrice de SPARC.

3. Procédé selon la revendication 1 ou 2, dans lequel le régime thérapeutique comprend en outre une radiothérapie.

4. Procédé selon la revendication 1, dans lequel l'agent chimiothérapeutique est sélectionné parmi le 5-fluorouracile, l'irinotécan, le cisplatine, la doxorubicine ou des combinaisons de ceux-ci.

5. Procédé selon la revendication 1, dans lequel une ou plusieurs mutations du promoteur de SPARC indiquent la résistance du cancer au régime thérapeutique.

6. Procédé selon l'une quelconque des revendications 1 à 5, comprenant en outre l'obtention d'informations sur le promoteur de SPARC pour le sujet.

7. Procédé selon l'une quelconque des revendications 1 à 6, dans lequel les une ou plusieurs mutations du promoteur de SPARC sont déterminées à l'aide d'un échantillon d'acide nucléique provenant du sujet.

8. Procédé selon la revendication 7, dans lequel l'échantillon d'acide nucléique a été obtenu à partir du suj et.

9. Procédé selon la revendication 8, dans lequel l'échantillon d'acide nucléique est issu d'un échantillon biologique provenant du cancer du sujet.

10. Procédé selon l'une quelconque des revendications 1 à 9, dans lequel ladite détermination de la présence ou de l'absence d'une ou plusieurs mutations du promoteur de SPARC dans le cancer du sujet est effectuée à l'aide d'une ou plusieurs des techniques suivantes :
(a) analyse de la longueur des fragments de restriction ;
(b) séquençage ;
(c) tests de microséquençage ;
(d) hybridation ;
(e) dosage INVADER ;
(f) tests d'hybridation de puces à ADN ;
(g) test de ligature d'oligonucléotides ;
(h) test à la 5' nucléase ;
(i) méthodes de relecture par la polymérase ;
(j) PCR allèle-spécifique ;
(k) spectroscopie de masse MALDI-TOF (Matrix Assisted Laser Desorption Inozation-Time Of Flight) ;
(l) test de la réaction en chaîne par ligase ;
(m) transduction électronique amplifiée par des enzymes ;
(n) test d'extension des paires de bases simples ; et
(o) lectures des données des séquences.

11. Procédé selon l'une quelconque des revendications 1 à 10, dans lequel le cancer est sélectionné parmi les suivants : carcinome ; mélanome ; sarcome ; lymphome et leucémie.

12. Procédé selon la revendication 11, dans lequel le carcinome est sélectionné parmi les suivants : carcinome de cellule squameuse ; adénocarcinome; carcinome à petites cellules ; carcinome à grandes cellules et combinaisons de ceux-ci.

13. Procédé selon la revendication 12, dans lequel le carcinome est dérivé d'une tumeur primaire située dans le tissu nasopharyngien, la tête et le cou, le poumon, l'oesophage, l'estomac, l'intestin, le rectum, le rein, l'appareil urinaire, la prostate, les testicules, l'ovaire, l'utérus, le col de l'utérus, le vagin, la peau ou les glandes endocrines.

14. Procédé selon la revendication 11, dans lequel le sarcome est sélectionné parmi les suivants : sarcome musculaire ; sarcome adipeux ; sarcome fibreux ; sarcome vasculaire et sarcome neural.

15. Procédé selon la revendication 11, dans lequel le lymphome est sélectionné parmi les suivants : maladie de Hodgkin et lymphome non hodgkinien.

16. Procédé selon la revendication 11, dans lequel la leucémie est sélectionnée parmi les suivantes : leucémie lymphoïde et leucémie myéloïde.

17. Procédé selon la revendication 11, dans lequel le cancer est un carcinome colorectal, un carcinome de la prostate, un carcinome ovarien ou un ostéosarcome.

18. Procédé selon l'une quelconque des revendications 1 à 17, dans lequel le cancer est un cancer inhibé par SPARC.

19. Procédé selon la revendication 18, dans lequel le cancer inhibé par SPARC est sélectionné parmi le carcinome colorectal, le carcinome de la prostate, le carcinome ovarien, l'ostéosarcome et le sarcome utérin.

20. Procédé de sélection d'un groupe de sujets pour déterminer l'efficacité d'un régime thérapeutique connu comme étant utile pour le traitement du cancer ou soupçonné de l'être, le procédé comprenant la détermination d'un génotype au niveau d'un ou plusieurs sites de mutation dans un promoteur de SPARC pour chaque sujet, dans lequel ledit génotype indique la sensibilité du sujet à un régime thérapeutique et dans lequel les une ou plusieurs mutations du promoteur de SPARC sont sélectionnées parmi les mutations suivantes relatives à SEQ N : 1 :
(a) 295delG et 301A>CC ;
(b) 315insA et 316G>A ;
(c) 341C>T, 342T>C, 344A>C, 347C>A, 354G>A, 356G>A, 359T>G et 363G>A ;
(d) 395C>T et 396A>T ;
(e) 488T>C ;
(f) 533A>G ;
(g) 734G>A ;
(h) 734delG ;
(i) 769T>A, 771G>C et 774G>C ;
(j) 784T>C, 786T>G et 788T>A ;
(k) 793T>G, 796T>A et 800T>C ;
(l) 820C>A et 824T>A;
(m) 864T>A et 867T>A ;
(n) 895T>G, 899T>A, 901G>A et 904G>A ;
(o) 901G>A;
(p) 901G>A, 904G>A, 929A>G et 934delC ;
(q) 922T>G, 928T>G et 935T>G ;
(r) 982T>C, 984T>G, 985G>A, 991G>A, 995G>A, 999C>T et 1000T>G ;
(s) 1063G>C ;
(t) 1079G>A ;
(u) 1085delC, 1086delA, 1087delG, 1089G>A, 1090T>C, 1091T>C et 1092G>T ;
(v) 1161C>A, 1162T>C, 1163delA et 1164delT ; et
(w) 1215C>T, 1216delG, 1217delG, 1218delG, 1219delG, 1220delT, 1221delG, 1222delG, 1223delA, 1224delG, 1225delG, 1226delG, 1227delG, 1228delA 1229delG, 1230delA, 1231delT, 1232delA, 1233delG, 1234delA, 1235delC, 1236delC et 1237C>T, et
dans lequel le régime thérapeutique est une chimiothérapie.

21. Procédé selon la revendication 20, comprenant en outre la détermination de la sensibilité de chaque sujet au régime thérapeutique et la comparaison de la réponse du sujet au régime thérapeutique sur la base du génotype du sujet.

22. Procédé selon la revendication 20, dans lequel la chimiothérapie est le 5-fluorouracile, l'irinotécan, le cisplatine, la doxorubicine ou des combinaisons de ceux-ci.

23. Procédé d'identification d'un sujet ayant une sensibilité accrue à une chimiothérapie pour le traitement d'un cancer chez un sujet qui en a besoin, comprenant l'étape de sélection d'une population de sujets pour identifier les suj ets qui présentent une mutation de leur séquence promotrice de SPARC, dans lequel l'identification d'un sujet présentant une mutation prédit la sensibilité réduite à la chimiothérapie, dans lequel la mutation du promoteur de SPARC est sélectionnée parmi une ou plusieurs des mutations suivantes relatives à SEQ N° : 1 :
(a) 295delG et 301A>CC ;
(b) 315insA et 316G>A ;
(c) 341C>T, 342T>C, 344A>C, 347C>A, 354G>A, 356G>A, 359T>G et 363G>A ;
(d) 395C>T et 396A>T ;
(e) 488T>C ;
(f) 533A>G ;
(g) 734G>A ;
(h) 734delG;
(i) 769T>A, 771G>C et 774G>C ;
(j) 784T>C, 786T>G et 788T>A ;
(k) 793T>G, 796T>A et 800T>C ;
(l) 820C>A et 824T>A ;
(m) 864T>A et 867T>a,
(n) 895T>G, 899T>A, 901G>A et 904G>A ;
(o) 901G>A ;
(p) 901G>A, 904G>A, 929A>G et 934delC ;
(q) 922T>G, 928T>G et 935T>G ;
(r) 982T>C, 984T>G, 985G>A, 991G>A, 995G>A, 999C>T et 1000T>G ;
(s) 1063G>C ;
(t) 1079G>A ;
(u) 1085delC, 1086delA, 1087delG, 1089G>A, 1090T>C, 1091T>C et 1092G>T ;
(v) 1161C>A, 1162T>C, 1163delA et 1164delT ; et
(w) 1215C>T, 1216delG, 1217delG, 1218delG, 1219delG, 1220delT, 1221delG, 1222delG, 1223delA, 1224delG, 1225delG, 1226delG, 1227delG, 1228delA, 1229delG, 1230delA 1231delT, 1232delA, 1233delG, 1234delA, 1235delC, 1236delC et 1237C>T.


### SEQUENCE LISTING

<110> The University of British Columbia
   TAI, Isabella T.
<120> SPARC PROMOTER MUTATIONS ASSOCIATED WITH DRUG RESISTANCE, AND METHODS, OLIGONUCLEOTIDES, CELLS AND ARRAYS ASSOCIATED THEREWITH
<130> 80021-835
<140> NOT YET ASSIGNED
   <141> 2005-11-30
<160> 55
<170> PatentIn version 3.3
<210> 1
   <211> 1370
   <212> DNA
   <213> Homo sapiens
<400> 1
   aatcccagca ctttgggagg ccgaggtggg tggatcatga ggtcaggcat tcgagaccag      60
   cctggccaac atagtgaaac cccatctcta ctaaaaatac aataaattag ctgggtgttg     120
   tggcatgtgc gcctgtaatc ccagctactc tggaggctga ggcgcgataa ttgcttgaac     180
   ccgggaggca gaggttgcag tgagccgaaa tcataccact gcactccagc ctgggcgaca     240
   gagtgagtga gactctgtct caaaacaaaa caaaacaaac aaacaaaaaa accggaaacc     300
   acaaaacttt ttgaggacaa ggaccagata tttattaatt ctcatacctc ccagagtgtt     360
   aggcacgaaa taaacattca accaagacct gttgcactga gcagttcata tataacagga     420
   gtgacccaag ttgaaacgta gaatcagccc tctcatacca ctttttggca ggtgatcata     480
   ggcaagttac ttagcatcta tgtttcctta ttattaaaat ggtcataatt acaatgccta     540
   agataagggg ttgctgtgaa gattattaaa tcctcagtaa actttggcta ttgttactcc     600
   tatgattatc atcaatatca tcacttacct tatctgttca atactggtgg cacaggtcca     660
   ccagctagat gtctaatccc ttatgtgtct attagtggta caagtggagt ttgagtggga     720
   tttttttttt taagaccagt tccaaatcat caaggatgat accactagta gcagcttgtc     780
   ttgtctgtac agtggtaagt cctggccttg cctttgtggc aaatacaacc cccttgaatt     840
   gcttggccct tctcagcatt gcctaatatt agggaggact cctgtaaagc tcactggtta     900
   gaagatcaag acacttgggc ctggttctac cccctggggg ccattgggta attccttgca     960
   gtctccaggc ctcacttgcc ctctgaacaa gaaagaggct gttctgggtc atccctccag    1020
   gcctgtccag ccctggcact ctgtgagtcg gtttaggcag cagccccgga acagatgagg    1080
   caggcagggt tgggacgttt ggtcaggaca gcccaccgca ccaagaggag gaaagaaatg    1140
   aaagacagag acagctttgg ctatgggaga aggaggaggc cgggggaagg aggagacagg    1200
   aggaggaggg accacggggt ggaggggaga tagacccagc ccagagctct gagtggtttc    1260
   ctgttgcctg tctctaaacc cctccacatt cccgcggtcc ttcagactgc ccggagagcg    1320
   cgctctgcct gccgcctgcc tgcctgccac tgagggttcc cagcaccatg               1370
<210> 2
   <211> 1370
   <212> DNA
   <213> Homo sapiens
<400> 2
   aatcccagca ctttgggagg ccgaggtggg tggatcatga ggtcaggcat tcgagaccag      60
   cctggccaac atagtgaaac cccatctcta ctaaaaatac aataaattag ctgggtgttg     120
   tggcatgtgc gcctgtaatc ccagctactc tggaggctga ggcgcgataa ttgcttgaac     180
   ccgggaggca gaggttgcag tgagccgaaa tcataccact gcactccagc ctgggcgaca     240
   gagtgagtga gactctgtct caaaacaaaa caaaacaaac aaacaaaaaa accgraamcc     300
   mcaaaacttt ttgaggacaa ggaccagata tttattaatt yycmtamctc ccarartgkt     360
   agrcacgaaa taaacattca accaagacct gttgywctga gcagttcata tataacagga     420
   gtgacccaag ttgaaacgta gaatcagccc tctcatacca ctttttggca ggtgatcata     480
   ggcaagtyac ttagcatcta tgtttcctta ttattaaaat ggtcataatt acratgccta     540
   agataagggg ttgctgtgaa gattattaaa tcctcagtaa actttggcta ttgttactcc     600
   tatgattatc atcaatatca tcacttacct tatctgttca atactggtgg cacaggtcca     660
   ccagctagat gtctaatccc ttatgtgtct attagtggta caagtggagt ttgagtggga     720
   tttttttttt taaraccagt tccaaatcat caaggatgat accactagwa scascttgtc     780
   ttgyckgwac agkggwaagy cctggccttg cctttgtggm aaawacaacc cccttgaatt     840
   gcttggccct tctcagcatt gccwaawatt agggaggact cctgtaaagc tcackggtwa     900
   raaratcaag acacttgggc ckggttckrc ccccKggggg ccattgggta attccttgca     960
   gtctccaggc ctcacttgcc cyckraacaa raaaraggyk gttctgggtc atccctccag    1020
   gcctgtccag ccctggcact ctgtgagtcg gtttaggcag casccccgga acagatgarg   1080
   caggcagggt tgggacgttt ggtcaggaca gcccaccgca ccaagaggag gaaagaaatg    1140
   aaagacagag acagctttgg myatgggaga aggaggaggc cgggggaagg aggagacagg    1200
   aggaggaggg accayggggt ggaggggaga tagacccagc ccagagctct gagtggtttc    1260
   ctgttgcctg tctctaaacc cctccacatt cccgcggtcc ttcagactgc ccggagagcg    1320
   cgctctgcct gccgcctgcc tgcctgccac tgagggttcc cagcaccatg               1370
<210> 3
   <211> 1370
   <212> DNA
   <213> Homo sapiens
<400> 3
   aatcccagca ctttgggagg ccgaggtggg tggatcatga ggtcaggcat tcgagaccag     60
   cctggccaac atagtgaaac cccatctcta ctaaaaatac aataaattag ctgggtgttg    120
   tggcatgtgc gcctgtaatc ccagctactc tggaggctga ggcgcgataa ttgcttgaac    180
   ccgggaggca gaggttgcag tgagccgaaa tcataccact gcactccagc ctgggcgaca    240
   gagtgagtga gactctgtct caaaacaaaa caaaacaaac aaacaaaaaa accgaaaccc    300
   ccaaaacttt ttgaggacaa ggaccagata tttattaatt ctcatacctc ccagagtgtt    360
   aggcacgaaa taaacattca accaagacct gttgcactga gcagttcata tataacagga    420
   gtgacccaag ttgaaacgta gaatcagccc tctcatacca ctttttggca ggtgatcata    480
   ggcaagttac ttagcatcta tgtttcctta ttattaaaat ggtcataatt acaatgccta    540
   agataagggg ttgctgtgaa gattattaaa tcctcagtaa actttggcta ttgttactcc    600
   tatgattatc atcaatatca tcacttacct tatctgttca atactggtgg cacaggtcca    660
   ccagctagat gtctaatccc ttatgtgtct attagtggta caagtggagt ttgagtggga    720
   tttttttttt taagaccagt tccaaatcat caaggatgat accactagta gcagcttgtc    780
   ttgtctgtac agtggtaagt cctggccttg cctttgtggc aaatacaacc cccttgaatt    840
   gcttggccct tctcagcatt gcctaatatt agggaggact cctgtaaagc tcactggtta    900
   gaagatcaag acacttgggc ctggttctac cccctggggg ccattgggta attccttgca    960
   gtctccaggc ctcacttgcc ccctgaacaa gaaagaggct gttctgggtc atccctccag   1020
   gcctgtccag ccctggcact ctgtgagtcg gtttaggcag cagccccgga acagatgagg   1080
   caggcagggt tgggacgttt ggtcaggaca gcccaccgca ccaagaggag gaaagaaatg   1140
   aaagacagag acagctttgg ctatgggaga aggaggaggc cgggggaagg aggagacagg   1200
   aggaggaggg accacggggt ggaggggaga tagacccagc ccagagctct gagtggtttc   1260
   ctgttgcctg tctctaaacc cctccacatt cccgcggtcc ttcagactgc ccggagagcg   1320
   cgctctgcct gccgcctgcc tgcctgccac tgagggttcc cagcaccatg              1370
<210> 4
   <211> 1371
   <212> DNA
   <213> Homo sapiens
<400> 4
   aatcccagca ctttgggagg ccgaggtggg tggatcatga ggtcaggcat tcgagaccag     60
   cctggccaac atagtgaaac cccatctcta ctaaaaatac aataaattag ctgggtgttg    120
   tggcatgtgc gcctgtaatc ccagctactc tggaggctga ggcgcgataa ttgcttgaac    180
   ccgggaggca gaggttgcag tgagccgaaa tcataccact gcactccagc ctgggcgaca    240
   gagtgagtga gactctgtct caaaacaaaa caaaacaaac aaacaaaaaa accggaaacc    300
   acaaaacttt ttgaagaaca aggaccagat atttattaat tctcatacct cccagagtgt    360
   taggcacgaa ataaacattc aaccaagacc tgttgcactg agcagttcat atataacagg    420
   agtgacccaa gttgaaacgt agaatcagcc ctctcatacc actttttggc aggtgatcat    480
   aggcaagtta cttagcatct atgtttcctt attattaaaa tggtcataat tacaatgcct    540
   aagataaggg gttgctgtga agattattaa atcctcagta aactttggct attgttactc    600
   ctatgattat catcaatatc atcacttacc ttatctgttc aatactggtg gcacaggtcc    660
   accagctaga tgtctaatcc cttatgtgtc tattagtggt acaagtggag tttgagtggg    720
   attttttttt ttaagaccag ttccaaatca tcaaggatga taccactagt agcagcttgt    780
   cttgtctgta cagtggtaag tcctggcctt gcctttgtgg caaatacaac ccccttgaat    840
   tgcttggccc ttctcagcat tgcctaatat tagggaggac tcctgtaaag ctcactggtt    900
   agaagatcaa gacacttggg cctggttcta ccccctgggg gccattgggt aattccttgc    960
   agtctccagg cctcacttgc cccctgaaca agaaagaggc tgttctgggt catccctcca   1020
   ggcctgtcca gccctggcac tctgtgagtc ggtttaggca gcagccccgg aacagatgag   1080
   gcaggcaggg ttgggacgtt tggtcaggac agcccaccgc accaagagga ggaaagaaat   1140
   gaaagacaga gacagctttg gctatgggag aaggaggagg ccgggggaag gaggagacag   1200
   gaggaggagg gaccacgggg tggaggggag atagacccag cccagagctc tgagtggttt   1260
   cctgttgcct gtctctaaac ccctccacat tcccgcggtc cttcagactg cccggagagc   1320
   gcgctctgcc tgccgcctgc ctgcctgcca ctgagggttc ccagcaccat g             1371
<210> 5
   <211> 1369
   <212> DNA
   <213> Homo sapiens
<400> 5
   aatcccagca ctttgggagg ccgaggtggg tggatcatga ggtcaggcat tcgagaccag     60
   cctggccaac atagtgaaac cccatctcta ctaaaaatac aataaattag ctgggtgttg    120
   tggcatgtgc gcctgtaatc ccagctactc tggaggctga ggcgcgataa ttgcttgaac    180
   ccgggaggca gaggttgcag tgagccgaaa tcataccact gcactccagc ctgggcgaca    240
   gagtgagtga gactctgtct caaaacaaaa caaaacaaac aaacaaaaaa accggaaacc    300
   acaaaacttt ttgaggacaa ggaccagata tttattaatt ctcatacctc ccagagtgtt    360
   aggcacgaaa taaacattca accaagacct gttgcactga gcagttcata tataacagga    420
   gtgacccaag ttgaaacgta gaatcagccc tctcatacca ctttttggca ggtgatcata    480
   ggcaagttac ttagcatcta tgtttcctta ttattaaaat ggtcataatt acaatgccta    540
   agataagggg ttgctgtgaa gattattaaa tcctcagtaa actttggcta ttgttactcc    600
   tatgattatc atcaatatca tcacttacct tatctgttca atactggtgg cacaggtcca    660
   ccagctagat gtctaatccc ttatgtgtct attagtggta caagtggagt ttgagtggga    720
   tttttttttt taaaccagtt ccaaatcatc aaggatgata ccactagtag cagcttgtct    780
   tgtctgtaca gtggtaagtc ctggccttgc ctttgtggca aatacaaccc ccttgaattg    840
   cttggccctt ctcagcattg cctaatatta gggaggactc ctgtaaagct cactggttag    900
   aagatcaaga cacttgggcc tggttctacc ccctgggggc cattgggtaa ttccttgcag    960
   tctccaggcc tcacttgccc cctgaacaag aaagaggctg ttctgggtca tccctccagg   1020
   cctgtccagc cctggcactc tgtgagtcgg tttaggcagc agccccggaa cagatgaggc   1080
   aggcagggtt gggacgtttg gtcaggacag cccaccgcac caagaggagg aaagaaatga   1140
   aagacagaga cagctttggc tatgggagaa ggaggaggcc gggggaagga ggagacagga   1200
   ggaggaggga ccacggggtg gaggggagat agacccagcc cagagctctg agtggtttcc   1260
   tgttgcctgt ctctaaaccc ctccacattc ccgcggtcct tcagactgcc cggagagcgc   1320
   gctctgcctg ccgcctgcct gcctgccact gagggttccc agcaccatg                1369
<210> 6
   <211> 1369
   <212> DNA
   <213> Homo sapiens
<400> 6
   aatcccagca ctttgggagg ccgaggtggg tggatcatga ggtcaggcat tcgagaccag      60
   cctggccaac atagtgaaac cccatctcta ctaaaaatac aataaattag ctgggtgttg     120
   tggcatgtgc gcctgtaatc ccagctactc tggaggctga ggcgcgataa ttgcttgaac     180
   ccgggaggca gaggttgcag tgagccgaaa tcataccact gcactccagc ctgggcgaca     240
   gagtgagtga gactctgtct caaaacaaaa caaaacaaac aaacaaaaaa accggaaacc     300
   acaaaacttt ttgaggacaa ggaccagata tttattaatt ctcatacctc ccagagtgtt     360
   aggcacgaaa taaacattca accaagacct gttgcactga gcagttcata tataacagga     420
   gtgacccaag ttgaaacgta gaatcagccc tctcatacca ctttttggca ggtgatcata     480
   ggcaagttac ttagcatcta tgtttcctta ttattaaaat ggtcataatt acaatgccta     540
   agataagggg ttgctgtgaa gattattaaa tcctcagtaa actttggcta ttgttactcc     600
   tatgattatc atcaatatca tcacttacct tatctgttca atactggtgg cacaggtcca     660
   ccagctagat gtctaatccc ttatgtgtct attagtggta caagtggagt ttgagtggga     720
   tttttttttt taagaccagt tccaaatcat caaggatgat accactagta gcagcttgtc     780
   ttgtctgtac agtggtaagt cctggccttg cctttgtggc aaatacaacc cccttgaatt     840
   gcttggccct tctcagcatt gcctaatatt agggaggact cctgtaaagc tcactggtta     900
   aaaaatcaag acacttgggc ctggttctgc ccctgggggc cattgggtaa ttccttgcag     960
   tctccaggcc tcacttgccc tctgaacaag aaagaggctg ttctgggtca tccctccagg    1020
   cctgtccagc cctggcactc tgtgagtcgg tttaggcagc agccccggaa cagatgaggc    1080
   aggcagggtt gggacgtttg gtcaggacag cccaccgcac caagaggagg aaagaaatga    1140
   aagacagaga cagctttggc tatgggagaa ggaggaggcc gggggaagga ggagacagga    1200
   ggaggaggga ccacggggtg gaggggagat agacccagcc cagagctctg agtggtttcc    1260
   tgttgcctgt ctctaaaccc ctccacattc ccgcggtcct tcagactgcc cggagagcgc    1320
   gctctgcctg ccgcctgcct gcctgccact gagggttccc agcaccatg                1369
<210> 7
   <211> 1367
   <212> DNA
   <213> Homo sapiens
<400> 7
   aatcccagca ctttgggagg ccgaggtggg tggatcatga ggtcaggcat tcgagaccag      60
   cctggccaac atagtgaaac cccatctcta ctaaaaatac aataaattag ctgggtgttg     120
   tggcatgtgc gcctgtaatc ccagctactc tggaggctga ggcgcgataa ttgcttgaac     180
   ccgggaggca gaggttgcag tgagccgaaa tcataccact gcactccagc ctgggcgaca     240
   gagtgagtga gactctgtct caaaacaaaa caaaacaaac aaacaaaaaa accggaaacc     300
   acaaaacttt ttgaggacaa ggaccagata tttattaatt ctcatacctc ccagagtgtt     360
   aggcacgaaa taaacattca accaagacct gttgcactga gcagttcata tataacagga     420
   gtgacccaag ttgaaacgta gaatcagccc tctcatacca ctttttggca ggtgatcata     480
   ggcaagttac ttagcatcta tgtttcctta ttattaaaat ggtcataatt acaatgccta     540
   agataagggg ttgctgtgaa gattattaaa tcctcagtaa actttggcta ttgttactcc     600
   tatgattatc atcaatatca tcacttacct tatctgttca atactggtgg cacaggtcca     660
   ccagctagat gtctaatccc ttatgtgtct attagtggta caagtggagt ttgagtggga     720
   tttttttttt taagaccagt tccaaatcat caaggatgat accactagta gcagcttgtc     780
   ttgtctgtac agtggtaagt cctggccttg cctttgtggc aaatacaacc cccttgaatt     840
   gcttggccct tctcagcatt gcctaatatt agggaggact cctgtaaagc tcactggtta     900
   gaagatcaag acacttgggc ctggttctac cccctggggg ccattgggta attccttgca     960
   gtctccaggc ctcacttgcc ctctgaacaa gaaagaggct gttctgggtc atccctccag    1020
   gcctgtccag ccctggcact ctgtgagtcg gtttaggcag cagccccgga acagatgagg    1080
   cagggacctg gacgtttggt caggacagcc caccgcacca agaggaggaa agaaatgaaa    1140
   gacagagaca gctttggcta tgggagaagg aggaggccgg gggaaggagg agacaggagg    1200
   aggagggacc acggggtgga ggggagatag acccagccca gagctctgag tggtttcctg    1260
   ttgcctgtct ctaaacccct ccacattccc gcggtccttc agactgcccg gagagcgcgc    1320
   tctgcctgcc gcctgcctgc ctgccactga gggttcccag caccatg                  1367
<210> 8
   <211> 1368
   <212> DNA
   <213> Homo sapiens
<400> 8
   aatcccagca ctttgggagg ccgaggtggg tggatcatga ggtcaggcat tcgagaccag     60
   cctggccaac atagtgaaac cccatctcta ctaaaaatac aataaattag ctgggtgttg    120
   tggcatgtgc gcctgtaatc ccagctactc tggaggctga ggcgcgataa ttgcttgaac    180
   ccgggaggca gaggttgcag tgagccgaaa tcataccact gcactccagc ctgggcgaca    240
   gagtgagtga gactctgtct caaaacaaaa caaaacaaac aaacaaaaaa accggaaacc    300
   acaaaacttt ttgaggacaa ggaccagata tttattaatt ctcatacctc ccagagtgtt    360
   aggcacgaaa taaacattca accaagacct gttgcactga gcagttcata tataacagga    420
   gtgacccaag ttgaaacgta gaatcagccc tctcatacca ctttttggca ggtgatcata    480
   ggcaagttac ttagcatcta tgtttcctta ttattaaaat ggtcataatt acaatgccta    540
   agataagggg ttgctgtgaa gattattaaa tcctcagtaa actttggcta ttgttactcc    600
   tatgattatc atcaatatca tcacttacct tatctgttca atactggtgg cacaggtcca    660
   ccagctagat gtctaatccc ttatgtgtct attagtggta caagtggagt ttgagtggga    720
   tttttttttt taagaccagt tccaaatcat caaggatgat accactagta gcagcttgtc    780
   ttgtctgtac agtggtaagt cctggccttg cctttgtggc aaatacaacc cccttgaatt    840
   gcttggccct tctcagcatt gcctaatatt agggaggact cctgtaaagc tcactggtta    900
   gaagatcaag acacttgggc ctggttctac cccctggggg ccattgggta attccttgca    960
   gtctccaggc ctcacttgcc ctctgaacaa gaaagaggct gttctgggtc atccctccag   1020
   gcctgtccag ccctggcact ctgtgagtcg gtttaggcag cagccccgga acagatgagg   1080
   caggcagggt tgggacgttt ggtcaggaca gcccaccgca ccaagaggag gaaagaaatg   1140
   aaagacagag acagctttgg acgggagaag gaggaggccg ggggaaggag gagacaggag   1200
   gaggagggac cacggggtgg aggggagata gacccagccc agagctctga gtggtttcct   1260
   gttgcctgtc tctaaacccc tccacattcc cgcggtcctt cagactgccc ggagagcgcg   1320
   ctctgcctgc cgcctgcctg cctgccactg agggttccca gcaccatg                1368
<210> 9
   <211> 2963
   <212> DNA
   <213> Homo sapiens
<400> 9
   gattgaattc cttgtacttt ttttcccttc tcagttctgc acttaactcg tctaaaaaaa 60
   ttaaaaaaga atttaagaaa ccacaaagct aagctgggtg cggtggctca cgcctgtaat 120
   cctagcactt tgggaagcca aggcattcgg attgcccaag ctcaggagtt cgagaccagc 180
   ctgggcaaca tgttgaaacc ccatttctac taaaaataca ataaattagc tgggtgttgt 240
   ggcatgtgcg cctgtaatcc cagctactct ggaggctgag gcgcgataat tgcttgaacc 300
   cgggaggcag aggttgcagt gagccgaaat cataccactg cactccagcc tgggcgacag 360
   agtgagtgag actctgtctc aaaacaaaac aaaacaaaca aacaaaaaaa ccggaaacca 420
   caaaactttt tgaggacaag gaccaggtat ttattaattc tcatacctcc cagagtgtta 480
   ggcacaaaat aaacattcaa ccaagacctg ttgcactgag cagttcatat ataacaggag 540
   tgacccaagt tgaaacgtag aatcagccct ctcataccac tttttgccag gtgatcatag 600
   gcaagttact tagcatctat gtttccttat tattaaaatg gtcataatta caatgcctaa 660
   gataaggggt tgctgtgaag attattaaat cctcagtaaa ctttggctat tgttactcct 720
   atgattatca tcaatatcat caattacctt atctgttcaa tactggtggc acaggtccac 780
   cagctagatg tctaatccct tatgtgtcta ttagtggtac aagtggagtt tgagtgggat 840
   tttttttttt aagaccagtt ccaaatcatc aaggatgata ccactagtag cagcttgtct 900
   tgtctgtaca gtggtaagtc ctggccttgc ctttgtggca aatacaaccc ccttgaattg 960
   cttggccctt ctcagcattg cctaatatta gggaggactc ctgtaaagct cactggttag 1020
   aagatcaaga cacttgggcc tggttctgcc cctgggggcc attgggtaat tccttgcagt 1080
   ctccaggcct cacttgccct ctgaacaaga aagaggctgt tctgggtcat ccctccaggc 1140
   ctgtccagcc ctggcactct gtgagtcggt ttaggcagca gccccggaac agatgaggca 1200
   ggcagggttg ggacgtttgg tcaggacagc ccaccgcaaa aagaggagga aagaaatgaa 1260
   agacagagac agctttggct atgggagaag gaggaggccg gggaaaggag gagacaggag 1320
   gaggagggac cacggggtgg aggggagata gacccagccc agagctctga gtggtttcct 1380
   gttgcctgtc tctaaacccc tccacattcc cgcggtcctt cagactgccc ggagagcgcg 1440
   ctctgcctgc cgcctgcctg cctgccactg agttaagaaa ccacaaagct aagctgggtg 1500
   cggtggctca cgcctgtaat cctagcactt tgggaagcca aggcattcgg attgcccaag 1560
   ctcaggagtt cgagaccagc ctgggcaaca tgttgaaacc ccatttctac taaaaataca 1620
   ataaattagc tgggtgttgt ggcatgtgcg cctgtaatcc cagctactct ggaggctgag 1680
   gcgcgataat tgcttgaacc cgggaggcag aggttgcagt gagccgaaat cataccactg 1740
   cactccagcc tgggcgacag agtgagtgag actctgtctc aaaacaaaac aaaacaaaca 1800
   aacaaaaaaa ccggaaacca caaaactttt tgaggacaag gaccaggtat ttattaattc 1860
   tcatacctcc cagagtgtta ggcacaaaat aaacattcaa ccaagacctg ttgcactgag 1920
   cagttcatat ataacaggag tgacccaagt tgaaacgtag aatcagccct ctcataccac 1980
   tttttgccag gtgatcatag gcaagttact tagcatctat gtttccttat tattaaaatg 2040
   gtcataatta caatgcctaa gataaggggt tgctgtgaag attattaaat cctcagtaaa 2100
   ctttggctat tgttactcct atgattatca tcaatatcat caattacctt atctgttcaa 2160
   tactggtggc acaggtccac cagctagatg tctaatccct tatgtgtcta ttagtggtac 2220
   aagtggagtt tgagtgggat tttttttttt aagaccagtt ccaaatcatc aaggatgata 2280
   ccactagtag cagcttgtct tgtctgtaca gtggtaagtc ctggccttgc ctttgtggca 2340
   aatacaaccc ccttgaattg cttggccctt ctcagcattg cctaatatta gggaggactc 2400
   ctgtaaagct cactggttag aagatcaaga cacttgggcc tggttctgcc cctgggggcc 2460
   attgggtaat tccttgcagt ctccaggcct cacttgccct ctgaacaaga aagaggctgt 2520
   tctgggtcat ccctccaggc ctgtccagcc ctggcactct gtgagtcggt ttaggcagca 2580
   gccccggaac agatgaggca ggcagggttg ggacgtttgg tcaggacagc ccaccgcaaa 2640
   aagaggagga aagaaatgaa agacagagac agctttggct atgggagaag gaggaggccg 2700
   gggaaaggag gagacaggag gaggagggac cacggggtgg aggggagata gacccagccc 2760
   agagctctga gtggtttcct gttgcctgtc tctaaacccc tccacattcc cgcggtcctt 2820
   cagactgccc ggagagcgcg ctctgcctgc cgcctgcctg cctgccactg aggtatgtgt 2880
   gacccccgcc cagcctttcc cttctatagt tgcaccaacc ccgacacccc cgttcacgcc 2940
   gtcagctcgt gtgcaaggga ggg 2963
<210> 10
   <211> 1349
   <212> DNA
   <213> Homo sapiens
<400> 10
   aatcccagca ctttgggagg ccgaggtggg tggatcatga ggtcaggcat tcgagaccag      60
   cctggccaac atagtgaaac cccatctcta ctaaaaatac aataaattag ctgggtgttg     120
   tggcatgtgc gcctgtaatc ccagctactc tggaggctga ggcgcgataa ttgcttgaac     180
   ccgggaggca gaggttgcag tgagccgaaa tcataccact gcactccagc ctgggcgaca     240
   gagtgagtga gactctgtct caaaacaaaa caaaacaaac aaacaaaaaa accggaaacc     300
   acaaaacttt ttgaggacaa ggaccagata tttattaatt ctcatacctc ccagagtgtt     360
   aggcacgaaa taaacattca accaagacct gttgcactga gcagttcata tataacagga     420
   gtgacccaag ttgaaacgta gaatcagccc tctcatacca ctttttggca ggtgatcata     480
   ggcaagttac ttagcatcta tgtttcctta ttattaaaat ggtcataatt acaatgccta     540
   agataagggg ttgctgtgaa gattattaaa tcctcagtaa actttggcta ttgttactcc     600
   tatgattatc atcaatatca tcacttacct tatctgttca atactggtgg cacaggtcca     660
   ccagctagat gtctaatccc ttatgtgtct attagtggta caagtggagt ttgagtggga     720
   tttttttttt taagaccagt tccaaatcat caaggatgat accactagta gcagcttgtc     780
   ttgtctgtac agtggtaagt cctggccttg cctttgtggc aaatacaacc cccttgaatt     840
   gcttggccct tctcagcatt gcctaatatt agggaggact cctgtaaagc tcactggtta     900
   gaagatcaag acacttgggc ctggttctac cccctggggg ccattgggta attccttgca     960
   gtctccaggc ctcacttgcc ctctgaacaa gaaagaggct gttctgggtc atccctccag    1020
   gcctgtccag ccctggcact ctgtgagtcg gtttaggcag cagccccgga acagatgagg    1080
   caggcagggt tgggacgttt ggtcaggaca gcccaccgca ccaagaggag gaaagaaatg    1140
   aaagacagag acagctttgg ctatgggaga aggaggaggc cgggggaagg aggagacagg    1200
   aggaggaggg accattagcc cagagctctg agtggtttcc tgttgcctgt ctctaaaccc    1260
   ctccacattc ccgcggtcct tcagactgcc cggagagcgc gctctgcctg ccgcctgcct    1320
   gcctgccact gagggttccc agcaccatg                                       1349
<210> 11
   <211> 399
   <212> DNA
   <213> Homo sapiens
<400> 11
   aataaattag ctgggtgttg tggcatgtgc gcctgtaatc ccagctactc tggaggctga      60
   ggcgcgataa ttgcttgaac ccgggaggca gaggttgcag tgagccgaaa tcataccact     120
   gcactccagc ctgggcgaca gagtgagtga gactctgtct caaaacaaaa caaaacaaac     180
   aaacaaaaaa accgaaaccc ccaaaacttt ttgaggacaa ggaccagata tttattaatt     240
   ctcatacctc ccagagtgtt aggcacgaaa taaacattca accaagacct gttgcactga     300
   gcagttcata tataacagga gtgacccaag ttgaaacgta gaatcagccc tctcatacca     360
   ctttttggca ggtgatcata ggcaagttac ttagcatct                            399
<210> 12
   <211> 407
   <212> DNA
   <213> Homo sapiens
<400> 12
   gtgttgtggc atgtgcgcct gtaatcccag ctactctgga ggctgaggcg cgataattgc      60
   ttgaacccgg gaggcagagg ttgcagtgag ccgaaatcat accactgcac tccagcctgg     120
   gcgacagagt gagtgagact ctgtctcaaa acaaaacaaa acaaacaaac aaaaaaaccg     180
   gaaaccacaa aactttttga agaacaagga ccagatattt attaattctc atacctccca     240
   gagtgttagg cacgaaataa acattcaacc aagacctgtt gcactgagca gttcatatat     300
   aacaggagtg acccaagttg aaacgtagaa tcagccctct cataccactt tttggcaggt     360
   gatcataggc aagttactta gcatctatgt ttccttatta ttaaaat                   407
<210> 13
   <211> 400
   <212> DNA
   <213> Homo sapiens
<400> 13
   ccagctactc tggaggctga ggcgcgataa ttgcttgaac ccgggaggca gaggttgcag     60
   tgagccgaaa tcataccact gcactccagc ctgggcgaca gagtgagtga gactctgtct    120
   caaaacaaaa caaaacaaac aaacaaaaaa accggaaacc acaaaacttt ttgaggacaa    180
   ggaccagata tttattaatt tccctaactc ccaaaatggt agacacgaaa taaacattca    240
   accaagacct gttgcactga gcagttcata tataacagga gtgacccaag ttgaaacgta    300
   gaatcagccc tctcatacca ctttttggca ggtgatcata ggcaagttac ttagcatcta    360
   tgtttcctta ttattaaaat ggtcataatt acaatgccta                          400
<210> 14
   <211> 406
   <212> DNA
   <213> Homo sapiens
<400> 14
   ttgcagtgag ccgaaatcat accactgcac tccagcctgg gcgacagagt gagtgagact     60
   ctgtctcaaa acaaaacaaa acaaacaaac aaaaaaaccg gaaaccacaa aactttttga    120
   ggacaaggac cagatattta ttaattctca tacctcccag agtgttaggc acgaaataaa    180
   cattcaacca agacctgttg ttctgagcag ttcatatata acaggagtga cccaagttga    240
   aacgtagaat cagccctctc ataccacttt ttggcaggtg atcataggca agttacttag    300
   catctatgtt tccttattat taaaatggtc ataattacaa tgcctaagat aaggggttgc    360
   tgtgaagatt attaaatcct cagtaaactt tggctattgt tactcc                   406
<210> 15
   <211> 403
   <212> DNA
   <213> Homo sapiens
<400> 15
   aaaaccggaa accacaaaac tttttgagga caaggaccag atatttatta attctcatac     60
   ctcccagagt gttaggcacg aaataaacat tcaaccaaga cctgttgcac tgagcagttc    120
   atatataaca ggagtgaccc aagttgaaac gtagaatcag ccctctcata ccactttttg    180
   gcaggtgatc ataggcaagt cacttagcat ctatgtttcc ttattattaa aatggtcata    240
   attacaatgc ctaagataag gggttgctgt gaagattatt aaatcctcag taaactttgg    300
   ctattgttac tcctatgatt atcatcaata tcatcactta ccttatctgt tcaatactgg    360
   tggcacaggt ccaccagcta gatgtctaat cccttatgtg tct                      403
<210> 16
   <211> 408
   <212> DNA
   <213> Homo sapiens
<400> 16
   tattaattct catacctccc agagtgttag gcacgaaata aacattcaac caagacctgt    60
   tgcactgagc agttcatata taacaggagt gacccaagtt gaaacgtaga atcagccctc   120
   tcataccact ttttggcagg tgatcatagg caagttactt agcatctatg tttccttatt   180
   attaaaatgg tcataattac gatgcctaag ataaggggtt gctgtgaaga ttattaaatc   240
   ctcagtaaac tttggctatt gttactccta tgattatcat caatatcatc acttacctta   300
   tctgttcaat actggtggca caggtccacc agctagatgt ctaatccctt atgtgtctat   360
   tagtggtaca agtggagttt gagtgggatt ttttttttta agaccagt                 408
<210> 17
   <211> 407
   <212> DNA
   <213> Homo sapiens
<400> 17
   atgcctaaga taaggggttg ctgtgaagat tattaaatcc tcagtaaact ttggctattg    60
   ttactcctat gattatcatc aatatcatca cttaccttat ctgttcaata ctggtggcac   120
   aggtccacca gctagatgtc taatccctta tgtgtctatt agtggtacaa gtggagtttg   180
   agtgggattt ttttttttaa aaccagttcc aaatcatcaa ggatgatacc actagtagca   240
   gcttgtcttg tctgtacagt ggtaagtcct ggccttgcct ttgtggcaaa tacaaccccc   300
   ttgaattgct tggcccttct cagcattgcc taatattagg gaggactcct gtaaagctca   360
   ctggttagaa gatcaagaca cttgggcctg gttctacccc ctggggg                   407
<210> 18
   <211> 406
   <212> DNA
   <213> Homo sapiens
<400> 18
   atgcctaaga taaggggttg ctgtgaagat tattaaatcc tcagtaaact ttggctattg    60
   ttactcctat gattatcatc aatatcatca cttaccttat ctgttcaata ctggtggcac   120
   aggtccacca gctagatgtc taatccctta tgtgtctatt agtggtacaa gtggagtttg   180
   agtgggattt ttttttttaa accagttcca aatcatcaag gatgatacca ctagtagcag   240
   cttgtcttgt ctgtacagtg gtaagtcctg gccttgcctt tgtggcaaat acaaccccct  300
   tgaattgctt ggcccttctc agcattgcct aatattaggg aggactcctg taaagctcac   360
   tggttagaag atcaagacac ttgggcctgg ttctaccccc tggggg                   406
<210> 19
   <211> 402
   <212> DNA
   <213> Homo sapiens
<400> 19
   aatcctcagt aaactttggc tattgttact cctatgatta tcatcaatat catcacttac    60
   cttatctgtt caatactggt ggcacaggtc caccagctag atgtctaatc ccttatgtgt   120
   ctattagtgg tacaagtgga gtttgagtgg gatttttttt tttaagacca gttccaaatc   180
   atcaaggatg ataccactag aaccaccttg tcttgtctgt acagtggtaa gtcctggcct   240
   tgcctttgtg gcaaatacaa cccccttgaa ttgcttggcc cttctcagca ttgcctaata   300
   ttagggagga ctcctgtaaa gctcactggt tagaagatca agacacttgg gcctggttct   360
   accccctggg ggccattggg taattccttg cagtctccag gc                       402
<210> 20
   <211> 407
   <212> DNA
   <213> Homo sapiens
<400> 20
   ttggctattg ttactcctat gattatcatc aatatcatca cttaccttat ctgttcaata    60
   ctggtggcac aggtccacca gctagatgtc taatccctta tgtgtctatt agtggtacaa   120
   gtggagtttg agtgggattt ttttttttaa gaccagttcc aaatcatcaa ggatgatacc   180
   actagtagca gcttgtcttg ccggaacagt ggtaagtcct ggccttgcct ttgtggcaaa   240
   tacaaccccc ttgaattgct tggcccttct cagcattgcc taatattagg gaggactcct   300
   gtaaagctca ctggttagaa gatcaagaca cttgggcctg gttctacccc ctgggggcca   360
   ttgggtaatt ccttgcagtc tccaggcctc acttgccctc tgaacaa                  407
<210> 21
   <211> 398
   <212> DNA
   <213> Homo sapiens
<400> 21
   gttactccta tgattatcat caatatcatc acttacctta tctgttcaat actggtggca    60
   caggtccacc agctagatgt ctaatccctt atgtgtctat tagtggtaca agtggagttt   120
   gagtgggatt ttttttttta agaccagttc caaatcatca aggatgatac cactagtagc   180
   agcttgtctt gtctgtacag gggaaagccc tggccttgcc tttgtggcaa atacaacccc   240
   cttgaattgc ttggcccttc tcagcattgc ctaatattag ggaggactcc tgtaaagctc   300
   actggttaga agatcaagac acttgggcct ggttctaccc cctgggggcc attgggtaat   360
   tccttgcagt ctccaggcct cacttgccct ctgaacaa                           398
<210> 22
   <211> 401
   <212> DNA
   <213> Homo sapiens
<400> 22
   atcacttacc ttatctgttc aatactggtg gcacaggtcc accagctaga tgtctaatcc    60
   cttatgtgtc tattagtggt acaagtggag tttgagtggg attttttttt ttaagaccag   120
   ttccaaatca tcaaggatga taccactagt agcagcttgt cttgtctgta cagtggtaag   180
   tcctggcctt gcctttgtgg aaaaaacaac ccccttgaat tgcttggccc ttctcagcat   240
   tgcctaatat tagggaggac tcctgtaaag ctcactggtt agaagatcaa gacacttggg   300
   cctggttcta ccccctgggg gccattgggt aattccttgc agtctccagg cctcacttgc   360
   cctctgaaca agaaagaggc tgttctgggt catccctcca g                       401
<210> 23
   <211> 397
   <212>. DNA
   <213> Homo sapiens
<400> 23
   gctagatgtc taatccctta tgtgtctatt agtggtacaa gtggagtttg agtgggattt    60
   ttttttttaa gaccagttcc aaatcatcaa ggatgatacc actagtagca gcttgtcttg   120
   tctgtacagt ggtaagtcct ggccttgcct ttgtggcaaa tacaaccccc ttgaattgct   180
   tggcccttct cagcattgcc aaaaattagg gaggactcct gtaaagctca ctggttagaa   240
   gatcaagaca cttgggcctg gttctacccc ctgggggcca ttgggtaatt ccttgcagtc   300
   tccaggcctc acttgccctc tgaacaagaa agaggctgtt ctgggtcatc cctccaggcc   360
   tgtccagccc tggcactctg tgagtcggtt taggcag                            397
<210> 24
   <211> 446
   <212> DNA
   <213> Homo sapiens
<400> 24
   gtggtacaag tggagtttga gtgggatttt tttttttaag accagttcca aatcatcaag 60
   gatgatacca ctagtagcag cttgtcttgt ctgtacagtg gtaagtcctg gccttgcctt 120
   tgtggcaaat acaaccccct tgaattgctt ggcccttctc agcattgcct aatattaggg 180
   aggactcctg taaagctcac gggtaaaaaa atcaagacac ttgggcctgg ttctaccccc 240
   tgggggccat tgggtaattc cttgcagtct ccaggcctca cttgccctct gaacaagaaa 300
   gaggctgttc tgggtcatcc ctccaggcct gtccagccct ggcactctgt gagtcggttt 360
   aggcagcagc cccggaacag atgaggcagg cagggttggg acgtttggtc aggacagccc 420
   accgcaccaa gaggaggaaa gaaatg 446
<210> 25
   <211> 440
   <212> DNA
   <213> Homo sapiens
<400> 25
   caagtggagt ttgagtggga tttttttttt taagaccagt tccaaatcat caaggatgat 60
   accactagta gcagcttgtc ttgtctgtac agtggtaagt cctggccttg cctttgtggc 120
   aaatacaacc cccttgaatt gcttggccct tctcagcatt gcctaatatt agggaggact 180
   cctgtaaagc tcactggtta aaagatcaag acacttgggc ctggttctac cccctggggg 240
   ccattgggta attccttgca gtctccaggc ctcacttgcc ctctgaacaa gaaagaggct 300
   gttctgggtc atccctccag gcctgtccag ccctggcact ctgtgagtcg gtttaggcag 360
   cagccccgga acagatgagg caggcagggt tgggacgttt ggtcaggaca gcccaccgca 420
   ccaagaggag gaaagaaatg 440
<210> 26
   <211> 439
   <212> DNA
   <213> Homo sapiens
<400> 26
   caagtggagt ttgagtggga tttttttttt taagaccagt tccaaatcat caaggatgat 60
   accactagta gcagcttgtc ttgtctgtac agtggtaagt cctggccttg cctttgtggc 120
   aaatacaacc cccttgaatt gcttggccct tctcagcatt gcctaatatt agggaggact 180
   cctgtaaagc tcactggtta aaaaatcaag acacttgggc ctggttctgc ccctgggggc 240
   cattgggtaa ttccttgcag tctccaggcc tcacttgccc tctgaacaag aaagaggctg 300
   ttctgggtca tccctccagg cctgtccagc cctggcactc tgtgagtcgg tttaggcagc 360
   agccccggaa cagatgaggc aggcagggtt gggacgtttg gtcaggacag cccaccgcac 420
   caagaggagg aaagaaatg 439
<210> 27
   <211> 419
   <212> DNA
   <213> Homo sapiens
<400> 27
   tttttttttt aagaccagtt ccaaatcatc aaggatgata ccactagtag cagcttgtct      60
   tgtctgtaca gtggtaagtc ctggccttgc ctttgtggca aatacaaccc ccttgaattg     120
   cttggccctt ctcagcattg cctaatatta gggaggactc ctgtaaagct cactggttag     180
   aagatcaaga cacttgggcc gggttcgacc cccggggggc cattgggtaa ttccttgcag      240
   tctccaggcc tcacttgccc tctgaacaag aaagaggctg ttctgggtca tccctccagg     300
   cctgtccagc cctggcactc tgtgagtcgg tttaggcagc agccccggaa cagatgaggc     360
   aggcagggtt gggacgtttg gtcaggacag cccaccgcac caagaggagg aaagaaatg      419
<210> 28
   <211> 419
   <212> DNA
   <213> Homo sapiens
<400> 28
   tgtctgtaca gtggtaagtc ctggccttgc ctttgtggca aatacaaccc ccttgaattg      60
   cttggccctt ctcagcattg cctaatatta gggaggactc ctgtaaagct cactggttag     120
   aagatcaaga cacttgggcc tggttctacc ccctgggggc cattgggtaa ttccttgcag     180
   tctccaggcc tcacttgccc ccgaaacaaa aaaaaggtgg ttctgggtca tccctccagg     240
   cctgtccagc cctggcactc tgtgagtcgg tttaggcagc agccccggaa cagatgaggc     300
   aggcagggtt gggacgtttg gtcaggacag cccaccgcac caagaggagg aaagaaatga     360
   aagacagaga cagctttggc tatgggagaa ggaggaggcc gggggaagga ggagacagg      419
<210> 29
   <211> 418
   <212> DNA
   <213> Homo sapiens
<400> 29
   ctaatattag ggaggactcc tgtaaagctc actggttaga agatcaagac acttgggcct      60
   ggttctaccc cctgggggcc attgggtaat tccttgcagt ctccaggcct cacttgccct     120
   ctgaacaaga aagaggctgt tctgggtcat ccctccaggc ctgtccagcc ctggcactct     180
   gtgagtcggt ttaggcagca cccccggaac agatgaggca ggcagggttg ggacgtttgg     240
   tcaggacagc ccaccgcacc aagaggagga aagaaatgaa agacagagac agctttggct     300
   atgggagaag gaggaggccg ggggaaggag gagacaggag gaggagggac cacggggtgg     360
   aggggagata gacccagccc agagctctga gtggtttcct gttgcctgtc tctaaacc       418
<210> 30
   <211> 402
   <212> DNA
   <213> Homo sapiens
<400> 30
   ctcctgtaaa gctcactggt tagaagatca agacacttgg gcctggttct accccctggg     60
   ggccattggg taattccttg cagtctccag gcctcacttg ccctctgaac aagaaagagg     120
   ctgttctggg tcatccctcc aggcctgtcc agccctggca ctctgtgagt cggtttaggc     180
   agcagccccg gaacagatga agcaggcagg gttgggacgt ttggtcagga cagcccaccg     240
   caccaagagg aggaaagaaa tgaaagacag agacagcttt ggctatggga gaaggaggag     300
   gccgggggaa ggaggagaca ggaggaggag ggaccacggg gtggagggga gatagaccca     360
   gcccagagct ctgagtggtt tcctgttgcc tgtctctaaa cc                        402
<210> 31
   <211> 415
   <212> DNA
   <213> Homo sapiens
<400> 31
   aaagctcact ggttagaaga tcaagacact tgggcctggt tctaccccct gggggccatt     60
   gggtaattcc ttgcagtctc caggcctcac ttgccccctg aacaagaaag aggctgttct    120
   gggtcatccc tccaggcctg tccagccctg gcactctgtg agtcggttta ggcagcagcc    180
   ccggaacaga tgaggcaggg acctggacgt ttggtcagga cagcccaccg caccaagagg    240
   aggaaagaaa tgaaagacag agacagcttt ggctatggga gaaggaggag gccgggggaa    300
   ggaggagaca ggaggaggag ggaccacggg gtggagggga gatagaccca gcccagagct    360
   ctgagtggtt tcctgttgcc tgtctctaaa cccctccaca ttcccgcggt ccttc          415
<210> 32
   <211> 408
   <212> DNA
   <213> Homo sapiens
<400> 32
   gtctccaggc ctcacttgcc ctctgaacaa gaaagaggct gttctgggtc atccctccag     60
   gcctgtccag ccctggcact ctgtgagtcg gtttaggcag cagccccgga acagatgagg    120
   caggcagggt tgggacgttt ggtcaggaca gcccaccgca ccaagaggag gaaagaaatg    180
   aaagacagag acagctttgg acgggagaag gaggaggccg ggggaaggag gagacaggag     240
   gaggagggac cacggggtgg aggggagata gacccagccc agagctctga gtggtttcct    300
   gttgcctgtc tctaaacccc tccacattcc cgcggtcctt cagactgccc ggagagcgcg    360
   ctctgcctgc cgcctgcctg cctgccactg agggttccca gcaccatg                 408
<210> 33
   <211> 339
   <212> DNA
   <213> Homo sapiens
<400> 33
   atccctccag gcctgtccag ccctggcact ctgtgagtcg gtttaggcag cagccccgga     60
   acagatgagg caggcagggt tgggacgttt ggtcaggaca gcccaccgca ccaagaggag    120
   gaaagaaatg aaagacagag acagctttgg ctatgggaga aggaggaggc cgggggaagg    180
   aggagacagg aggaggaggg accattagcc cagagctctg agtggtttcc tgttgcctgt    240
   ctctaaaccc ctccacattc ccgcggtcct tcagactgcc cggagagcgc gctctgcctg    300
   ccgcctgcct gcctgccact gagggttccc agcaccatg                           339
<210> 34
   <211> 22
   <212> DNA
<213> Artificial sequence
<220>
   <223> Primer
<400> 34
   ttctgcactt aactcgtcta aa 22
<210> 35
   <211> 22
   <212> DNA
   <213> Artificial sequence
<220>
   <223> Primer
<400> 35
   gctgattcta cgtttcaact tg 22
<210> 36
   <211> 22
   <212> DNA
   <213> Artificial sequence
<220>
   <223> Primer
<400> 36
   tgaggacaag gaccaggtat tt 22
<210> 37 <211> 21
   <212> DNA
   <213> Artificial sequence
<220>
   <223> Primer
<400> 37
   aggaaaccac tcagagctct g 21
<210> 38
   <211> 16
   <212> DNA
   <213> Artificial sequence
<220>
   <223> Primer
<400> 38
   gtcatccctc caggcc 16
<210> 39
   <211> 19
   <212> DNA
   <213> Artificial sequence
<220>
   <223> Primer
<400> 39
   cggggttggt gcaactata 19
<210> 40
   <211> 40
   <212> DNA
   <213> Artificial sequence
<220>
   <223> Primer
<400> 40
   tgtaaaacga cggccagttg aggacaagga ccaggtattt 40
<210> 41
   <211> 38
   <212> DNA
   <213> Artificial sequence
<220>
   <223> Primer
<400> 41
   caggaaacag ctatgacagg aaaccactca gagctctg 38
<210> 42
   <211> 37
   <212> DNA
   <213> Artificial sequence
<220>
   <223> Primer
<400> 42
   tgtaaaacga cggccagtgg tggaggggag atagacc 37
<210> 43
   <211> 37
   <212> DNA
   <213> Artificial sequence
<220>
   <223> Primer
<400> 43
   caggaaacag ctatgactcc caaggaactc aggacag 37
<210> 44
   <211> 40
   <212> DNA
   <213> Artificial sequence
<220>
   <223> Primer
<400> 44
   tgtaaaacga cggccagttg aggacaagga ccaggtattt 40
<210> 45
   <211> 38
   <212> DNA
<213> Artificial sequence
<220>
   <223> Primer
<400> 45
   caggaaacag ctatgacagg aaaccactca gagctctg 38
<210> 46
   <211> 38
   <212> DNA
   <213> Artificial sequence
<220>
   <223> Primer
<400> 46
   tgtaaaacga cggccagtaa atggcaccat cacaacct 38
<210> 47
   <211> 37
   <212> DNA
   <213> Artificial sequence
<220>
   <223> Primer
<400> 47
   caggaaacag ctatgacccg aagcctgtgt cctactt 37
<210> 48
   <211> 40
   <212> DNA
   <213> Artificial sequence
<220>
   <223> Primer
<400> 48
   tgtaaaacga cggccagttt ctgcacttaa ctcgtctaaa 40
<210> 49
   <211> 39
   <212> DNA
   <213> Artificial sequence
<220>
   <223> Primer
<400> 49
   caggaaacag ctatgacgct gattctacgt ttcaacttg 39
<210> 50
   <211> 34
   <212> DNA
   <213> Artificial sequence
<220>
   <223> Primer
<400> 50
   tgtaaaacga cggccagtgt catccctcca ggcc 34
<210> 51
   <211> 36
   <212> DNA
   <213> Artificial sequence
<220>

   <223> Primer
<400> 51
   caggaaacag ctatgaccgg ggttggtgca actata 36
<210> 52
   <211> 23
   <212> DNA
   <213> Artificial sequence
<220>
   <223> Primer
<400> 52
   cgaagaggag gtggtggcgg aaa 23
<210> 53
   <211> 26
   <212> DNA
   <213> Artificial sequence
<220>
   <223> Primer
<400> 53
   ggttgttgtc ctcatccctc tcatac 26
<210> 54
   <211> 24
   <212> DNA
   <213> Artificial sequence
<220>
   <223> Primer
<400> 54
   ctctctgctc ctcctgttcg acag 24
<210> 55
   <211> 24
   <212> DNA
   <213> Artificial sequence
<220>
   <223> Primer
<400> 55
   aggggtctta ctccttggag gcca 24